# EUROPEAN PATENT APPLICATION

(11) **EP 2 014 302 A1**
(43) Date of publication of application: **14.01.2009**
(21) Application number: 07290881.7
(22) Date of filing: 12.07.2007
(51) Int. Cl.: A61K 39/395, A61P 35/00, C07K 16/28, C07K 16/30, G01N 33/577

(54) **An antibody specific for the Tn antigen for the treatment of cancer**

(71) Applicant: Institut Curie, 75248 Paris Cedex 05 (FR); INSTITUT NATIONAL DE LA SANTE ET DE LA RECHERCHE MEDICALE (INSERM), 75013 Paris (FR)
(72) Inventor: Pritsch, Otto, 11200 Montevideo (UY); Oppezzo, Pablo, 15006 Montevideo (UY); Perez, Franck, 75013 Paris (FR); Moutel, Sandrine, 75013 Paris (FR); Hubert-Haddad, Pascal, 92330 Sceaux (FR); Amigorena, Sebastian, 75013 Paris (FR); Sastre, Xavier, 94300 Vincennes (FR); Osinaga, Eduardo, 11400 Montevideo (UY)
(74) Representative: Colombet, Alain André

(57) **Abstract**

The application provides a pharmaceutical composition comprising an antibody, directed against Tn antigen which comprises CDRs derived from 83D4 monoclonal antibody or an immunoconjugate of said antibody, said pharmaceutical composition being intended for the treatment of cancer.

## Description

The instant invention provides a pharmaceutical composition comprising an antibody directed against Tn antigen which comprises CDRs derived from 83D4 monoclonal antibody, or an immunoconjugate of said antibody, said pharmaceutical composition being intended for the treatment of cancer.

Tumor cells are derived from the malignant transformation of normal cells, and thus are formed in majority by self proteins not recognized by the immune system. However, the malignant phenotype is often accompanied by changes in antigenicity, and tumor cells have been demonstrated to express antigens that can be recognized by CTL. These so-called tumor-associated antigens can be targeted for immunotherapy and generate an immune response able to eradicate the tumor (Van den Eynde, Current Opin. Immunol., 1997, 9(5):684-93; Gilboa, Immunity, 1999, 11 (3):263-70). They have been classified into several groups, according to the mechanisms by which they are generated. The first one represents patient-specific neoantigens, generated by somatic mutations occurring in normal genes because of the genetic instability of tumor cells. The second group corresponds to tumor-specific antigens not expressed in normal tissues, due to mutations appearing subsequently to the oncogenic process, and are shared among patients with the same type of tumor. The antigens of the third group are shared silent antigens reactivated in various types of tumor cells. Finally, the last group concerns differentiation antigens expressed by both normal and tumor cells. Some tumor-associated antigens are expressed at the plasma membrane with sufficient selectivity to distinguish between tumor and normal cells and thus can be targeted by monoclonal antibodies for immunotherapy (Christiansen, Mol Cancer Ther, 2004, 3(11):1493-501).

Monoclonal antibodies (mAb) recognizing tumor-cell surface antigens have been proven to be efficient at eradicating cancers in animal models. Several mAbs are now successfully used in the course of treatment of patients bearing some types of haematopoietic or solid tumors (Von Mehren et al., Annu Rev Med, 2003, 54:343-369, Harris, Lancet Oncol, 2004, 5: 292-302). The most commonly used mAbs are those directed against growth factor receptors such as Her2/neu (i.e. Herceptin used in breast cancers) and the EGF receptor (i.e. Cetuximab used in colon carcinomas). MAbs specific for differentiation antigens not expressed on progenitor haematopoietic cells are also used, such as the anti-CD20 mAb Rituximab (used in lymphomas), the anti-CD33 mAb Gemtuzumab (used in acute myeloid leukemia) and the anti-CD52 mAb Alemtuzumab (used in B-cell chronic lymphocytic leukaemia).

Their mechanism of action may vary according to the mAb considered. Indeed, *in vitro,* mAb can block growth factor receptor binding, or induce direct apoptosis, or can kill tumor cells indirectly by antibody-dependent-cellular cytotoxicity (ADCC) or complement dependent cellular (CDC). Nevertheless, the mechanisms of tumor growth inhibition *in vivo* (either in animal models or in patients) remain still elusive, and cannot be predicted directly by the results obtained *in vitro.* Indeed, expression of an antigen at the cell surface is not sufficient to predict that a specific mAb directed against said antigen would induce ADCC. This is the case of Herceptin which can induce ADCC in cells displaying an amplification of Her2, but not in normal cells expressing Her2 at a low level (Carter, PNAS, 1992, 89: 4285-4289). Moreover, no direct demonstration that ADCC could occur *in vivo* has ever been reported: only a greater infiltration of the tumor samples by lymphoid cells has been observed in patients treated with Herceptin compared to untreated ones (Gennari, Clin Cancer Res, 2004, 10:5650-5655).

Such therapeutic mAbs are particularly interesting because of their high tumor specificity and minor binding and toxicity toward normal cells, contrary to conventional anti-cancer therapies. However, only few tumor-associated antigens with high tumor specificity and low expression on normal cells are available (Christiansen et al., Mol Cancer Ther, 2004, 11:1493-1501). Thus, the search for and the development of new tumor-specific mAbs is a promising and increasing field of investigation, mainly for tumors for which few effective therapies are available, such as epithelial cancers.

Some oligosaccharide structures such as CA19.9 (sialyl Lewis A), the blood-group antigens Lewis A, sialyl Lewis C, sialyl Lewis X, and the carbohydrate epitopes Tn and sialyl-Tn are highly overexpressed by epithelial tumor cells (Hollingsworth, Nature Rev Cancer, 2004, 4(1):45-60). The glyco-peptidic epitopes Tn and sialyl-Tn are cryptic determinants found in mucins and mucin-type glycoproteins which are masked by other sugar residues in normal cells. However, aberrant protein glycosylation processes occur in tumor cells, leading to the exposure of these antigens in about 90% of human carcinomas. Thus, they are some of the most specific tumor-associated antigens described so far, and the intensity of their expression is correlated to the aggressiveness of the tumors (Springer, Science, 1984, 224:1198-1206). Tn is formed by a N-acetyl galactosamine residue (GalNac) linked by O-glycosylation to serine or threonine amino-acids present in the backbone of mucin-type glycoproteins. Moreover, Tn expression appears in different types of human epithelial carcinomas, contrary to the antigens recognized by the yet commercially available therapeutic mAbs which target only one or few types of tissues.

Several anti-Tn mAbs have been reported yet. Ohshio, et al. (J Cancer Res Clin Oncol, 1995, 121:247-252), Avichezer et al. (Int J Cancer, 1997, 72:119-127) described mouse anti-Tn mAb reactive with epithelial carcinomas, but no data concerning any effect of these mAb on tumor growth in vivo are available. Recently, Schietinger et al. (Science, 2006, 314:304-308) published a murine mAb recognizing GalNac in the context of the peptide 75-84 of the murine protein OTS8. This mAb is able to reduce the growth of a murine sarcoma in mice, but its activity was restricted to this particular tumor displaying a mutation in the chaperone protein Cosmc together with the expression of the OTS8 protein. Indeed, this mAb did not bind to Jurkat cells, described to express Tn and a mutated form of Cosmc (Ju, PNAS, 2002, 99:16613-16618).

In addition, it has been recently shown that injection of a synthetic Tn antigen to tumor-bearing mice inhibits the growth of a murine breast carcinoma, and induces anti-Tn antibodies. This polyclonal anti-Tn response is able to kill tumor cells by ADCC *in vitro,* but is not demonstrated to be responsible for the reduction of tumor growth *in vivo* (Lo-Man, J Immunol, 2001, 166 : 2849-2854; Lo-Man, Cancer Res, 2004, 64 :4987-4994).

However, although mAbs specific for the Tn antigen may be interesting to be tested in passive immunotherapy, it remains unpredictable whether anti-Tn mAb may have an inhibitory effect *in vivo.* Indeed, first, Tn expression in tumor cells has been evidenced by immunohistochemistry, and only very few data are available reporting the recognition of Tn by mAbs at the tumor cell surface by flow cytometry (Lo-Man, Cancer Res., 1999, 59:1520-1524). In addition, Tn can be expressed on mucins and mucin-type proteins, which can transduce various positive and negative signals to the cell. Finally, as discussed above, the expression of an antigen at the cell surface is not sufficient to predict that cell death will occur *in vivo,* either directly or by ADCC.

The murine mAb 83D4 was originally produced by immunisation with permeabilised cell suspensions obtained from formalin-fixed paraffin block sections of human breast carcinoma (Pancino, Hybridoma, 1990, 9:389-395). The antigen recognized by the 83D4 monoclonal antibody was later identified as the Tn antigen.

The mAb 83D4 was further used to detect Tn antigen by immunohistochemistry in rat chemically-induced tumors (Babino et al., Int. J. Cancer, 2000, 86: 753-759; Berriel et al., Oncology Reports, 2005, 14:219-227).

The variable region of mAb 83D4 was cloned and sequenced, and a scFv fragment having Tn-binding activity was constructed (Babino et al., Hybridoma, 1997, 16(4),317-324).

Two chimeric anti-Tn (chi-83D4) mAb were constructed by fusion of the variable fragments of the murine mAb 83D4 to the constant region of human IgMk and lgG1k (Oppezzo, Hybridoma, 2000, 19:229-239). The chimeric lgMk and lgG1k were found to bind to permeabilised breast carcinoma MCF7 cells expressing the Tn antigen.

Furthermore, patent US 6,365,124 describes a peptide having binding specificity for the Tn antigen which consists of amino acids 20 to 135 of 83D4 VH (GenPept AAG02617), or fragments thereof of at least 10 amino acids. The detectably labeled peptide is used to detect the presence of shed tumor antigen in lymphoid tissues involved in pro-tumor immune response. Thus, the peptide is only used for detection purposes. The therapeutic treatment described in patent US 6,365,124 consists of surgical resection of the lymphoid tissues detected.

The Inventors have established now that the chi-83D4 mAb specifically recognises cell surface Tn antigen in a panel of human epithelial tumor cell lines. They further demonstrated that the chi-83D4 mAb efficiently inhibits the growth of human carcinomas in mouse xenograft models.

As the Tn antigen is widely expressed on tumor cells, the invention thus provides a method of treating and/or preventing cancer based on the chimeric mouse/human chi-83D4 monoclonal antibody.

### Definitions

As used herein, "Tn antigen" denotes GalNAcα-O-Ser/Thr, i.e. an antigen wherein the GalNAc residue is alpha-linked directly to the hydroxyl group of a serine or threonine residue of a polypeptide chain expressed intracellularly or at the cell surface.

"83D4" is meant for the murine mAb which was originally described in Pancino, Hybridoma, 1990, 9:389-395. "chi-83D4" denotes chimeric anti-Tn mAbs which were constructed by fusion of the variable fragments of the murine mAb 83D4 to the constant region of human IgMk and IgG1K (Oppezzo, Hybridoma, 2000, 19:229-239).

The terms "antibody" and "immunoglobulin" have the same meaning and are used indifferently in the present invention. Antibody refers to immunoglobulin molecules and immunologically active portions of immunoglobulin molecules, i.e., molecules that contain an antigen binding site that immunospecifically binds an antigen. As such, the term antibody encompasses not only whole antibody molecules, but also antibody fragments as well as variants (including derivatives) of antibodies and antibody fragments. In natural antibodies, two heavy chains are linked to each other by disulfide bonds and each heavy chain is linked to a light chain by a disulfide bond. There are two types of light chain, lambda (A) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non hypervariable or framework regions (FR) influence the overall domain structure and hence the combining site. Complementarity determining regions (CDRs) refer to amino acid sequences which, together, define the binding affinity and specificity of the natural Fv region of a native immunoglobulin binding-site. The light and heavy chains of an immunoglobulin each have three CDRs, designated L-CDR1, L-CDR2, L-CDR3 and H-CDR1, H-CDR2, H-CDR3, respectively. Therefore, an antigen-binding site includes six CDRs, comprising the CDR set from each of a heavy and a light chain V region.

Framework Regions (FRs) refer to amino acid sequences interposed between CDRs, i.e. to those portions of immunoglobulin light and heavy chain variable regions that are relatively conserved among different immunoglobulins in a single species, as defined by Kabat, et al (Sequences of Proteins of Immunological Interest (National Institutes of Health, Bethesda, Md., 1991). As used herein, a "human framework region" is a framework region that is substantially identical (about 85%, or more, in particular 90%, 95%, or 100%) to the framework region of a naturally occurring human antibody.

The term "monoclonal antibody" or "mAb" as used herein refers to an antibody molecule of a single amino acid composition, that is directed against a specific antigen and that is produced by a single clone of B cells or hybridoma.

The term "chimeric antibody" refers to an engineered antibody which comprises a VH domain and a VL domain of an antibody derived from a non-human animal, in association with a CH domain and a CL domain of another antibody, in particular a human antibody. As the non-human animal, any animal such as mouse, rat, hamster, rabbit or the like can be used.

The term "humanized antibody" refers to antibodies in which the framework or "complementarity determining regions" (CDR) have been modified to comprise the CDR from a donor immunoglobulin of different specificity as compared to that of the parent immunoglobulin. In a preferred embodiment, a mouse CDR is grafted into the framework region of a human antibody to prepare the "humanized antibody".

"Antibody fragments" comprise a portion of an intact antibody, preferably the antigen binding or variable region of the intact antibody. Examples of antibody fragments include Fv, Fab, F(ab')2, Fab', dsFv, scFv, sc(Fv)2, diabodies and multispecific antibodies formed from antibody fragments.

The term "Fab" denotes an antibody fragment having a molecular weight of about 50,000 and antigen binding activity, in which about a half of the N-terminal side of H chain and the entire L chain, among fragments obtained by treating IgG with a protease, papaine, are bound together through a disulfide bond.

The term "F(ab')₂" refers to an antibody fragment having a molecular weight of about 100,000 and antigen binding activity, which is slightly larger than the Fab bound via a disulfide bond of the hinge region, among fragments obtained by treating IgG with a protease, pepsin.

The term "Fab"' refers to an antibody fragment having a molecular weight of about 50,000 and antigen binding activity, which is obtained by cutting a disulfide bond of the hinge region of the F(ab')₂.

A single chain Fv ("scFv") polypeptide is a covalently linked VH::VL heterodimer which is usually expressed from a gene fusion including VH and VL encoding genes linked by a peptide-encoding linker. The human scFv fragment of the invention includes CDRs that are held in appropriate conformation, preferably by using gene recombination techniques. "dsFv" is a VH::VL heterodimer stabilised by a disulphide bond. Divalent and multivalent antibody fragments can form either spontaneously by association of monovalent scFvs, or can be generated by coupling monovalent scFvs by a peptide linker, such as divalent sc(Fv)₂.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

### Anti-Tn monoclonal antibodies

According to the invention, an antibody directed against Tn antigen comprises a variable light (VL) chain which comprises L-CDR1, L-CDR2, and L-CDR3 of the immunoglobulin light chain shown in SEQ ID NO:8 and/or a variable heavy (VH) chain which comprises H-CDR1, H-CDR2, and H-CDR3 of the immunoglobulin heavy chain shown in SEQ ID NO:6.

In the heavy chain, the VH region is encoded by nucleotides 1 to 405 of SEQ ID NO:5, and its includes the CDR1 (bp 148 to 162), the CDR2 (bp 205 to 255) and the CDR3 (bp 352 tp 372) fragments. The Fc region (bp 406 to 1395) includes the CH1 (bp 406 to 699), the CH2 (bp 745 to 1074) and the CH3 (bp 1075 to 1395) fragments.

In the light chain, the VL region (bp 1 to 381 of SEQ ID NO:7) includes the CDR1 (bp 130 to 162), the CDR2 (bp 208 to 228) and the CDR3 (bp 325 to 351) fragments. The C kappa region is encode by nucleotides 383 to 702 of SEQ ID NO:7.

The sequences of L-CDR1, L-CDR2, and L-CDR3 are respectively RASQNIGTSIH (SEQ ID NO:25), YASESVS (SEQ ID NO:26) and QHTNSWPTT (SEQ ID NO:27).

The sequences of H-CDR1, H-CDR2, and H-CDR3 are respectively DHAIH (SEQ ID NO:28), YFSPGNGDIKYNEKFKG (SEQ ID NO:29) and SYGNYDY (SEQ ID NO:30).

These CDRS are derived from mouse 83D4 monoclonal antibody (mAb), and are found in chimeric chi-83D4.

More specifically, the antibody may comprise (i) the variable region of the light chain shown in SEQ ID NO:8 (amino acids in positions 1 to 127 of SEQ ID NO:8) or an amino acid sequence at least 85%, preferably 90%, more preferably 95%, still preferably 98%, identical to amino acids in positions 1 to 127 of SEQ ID NO:8, and/or (ii) the variable region of the heavy chain shown in SEQ ID NO:6 (amino acids in positions 1 to 135 of SEQ ID NO:6) or an amino acid sequence at least 85%, preferably 90%, more preferably 95%, still preferably 98%, identical to amino acids in positions 1 to 135 of SEQ ID NO:6.

The antibody may be a whole antibody molecule, i.e. it further comprises, fused to said VH and VL chains, immunoglobulin constant regions of heavy (CH) and light chains (CL). In particular, said antibody may be a monoclonal antibody.

According to an embodiment, the antibody is a chimeric antibody. Thus, preferably said antibody further comprises human constant regions of heavy (CH) and light chains (CL).

In particular, an antibody according to the invention may comprise the light chain shown in SEQ ID NO:8 or an amino acid sequence at least 85%, preferably 90%, more preferably 95%, still preferably 98%, identical to SEQ ID NO:8. An antibody according to the invention may also comprise the heavy chain shown in SEQ ID NO:6 or an amino acid sequence at least 85%, preferably 90%, more preferably 95%, still preferably 98%, identical to SEQ ID NO:6. Preferably, an antibody according to the invention comprises (i) the light chain shown in SEQ ID NO:8 or an amino acid sequence at least 85%, preferably 90%, more preferably 95%, still preferably 98%, identical to SEQ ID NO:8 and (ii) the heavy chain shown in SEQ ID NO:6 or an amino acid sequence at least 85%, preferably 90%, more preferably 95%, still preferably 98%, identical to SEQ ID NO:6.

The antibody according to the invention may also be a humanised antibody, i.e. it contains the L-CDR1, L-CDR2, and L-CDR3 of the light chain shown in SEQ ID NO:8 and /or the H-CDR1, H-CDR2, and H-CDR3 of the heavy chain shown in SEQ ID NO:6 inserted into human framework regions. Where the antibody is a whole antibody molecule, it further comprises human constant regions of heavy (CH) and light chains (CL).

The antibody according to the invention may be an antibody fragment.

In particular, the inventors have developed an scFV comprising amino acids 1 to 244 of SEQ ID NO:24 and which behaves like the parent 83D4 antibody and specifically detects the Tn antigen present at the surface of Jurkat cells.

Accordingly, the invention provides an antibody, more particularly a 83D4 scFV, directed against Tn antigen which comprises, or consists of, amino acids 1 to 244 of SEQ ID NO:24, or an amino acid sequence at least 85%, preferably 90%, more preferably 95%, more preferably 97%, still preferably 99%, identical to amino acid 1 to 244 of SEQ ID NO:24.

Sequence identity over a defined length of amino acid sequences may be identified by alignment using, for example, the GCG (Genetics Computer Group, Program Manual for the GCG Package, Version 7, Madison, Wisconsin) pileup program, or any of alignment tool publicly available such as FASTA, BLAST, Needleman-Wunsch global alignment or Smith-Waterman local alignment.

Another object of the invention is an antibody, in particular a scFv, directed against Tn antigen which comprises, or consist of, (i) amino acids 1 to 244 of SEQ ID NO:24, or (ii) an amino acid sequence at least 85%, preferably 90%, more preferably 95%, more preferably 97%, still preferably 99%, identical to amino acid 1 to 244 of SEQ ID NO:24 provided said amino acid sequence comprises L-CDR1, L-CDR2, and L-CDR3 of the light chain shown in SEQ ID NO:8 and H-CDR1, H-CDR2, and H-CDR3 of the heavy chain shown in SEQ ID NO:6.

The invention also provides a 83D4scFv-hFc2 antibody which comprise the 83D4 scFV fused to a Fc region of a human IgG2 comprising the CH2 and CH3 domains of the heavy chain and the Hinge region.

Thus the invention relates to an antibody directed against Tn antigen which comprises, or consist of, (i) the amino acid sequence of SEQ ID NO:24, or (ii) an amino acid sequence at least 85%, preferably 90%, more preferably 95%, more preferably 97%, still preferably 99%, identical to SEQ ID NO:24 provided said amino acid sequence comprises L-CDR1, L-CDR2, and L-CDR3 of the light chain shown in SEQ ID NO:8 and H-CDR1, H-CDR2, and H-CDR3 of the heavy chain shown in SEQ ID NO:6.

### Methods of producing antibodies of the invention

Antibodies according to invention may be produced by any technique known in the art, such as, without limitation, any chemical, biological, genetic or enzymatic technique, either alone or in combination.

Knowing the amino acid sequence of the desired sequence, one skilled in the art can readily produce said antibodies, by standard techniques for production of polypeptides. For instance, they can be synthesized using well-known solid phase method, preferably using a commercially available peptide synthesis apparatus (such as that made by Applied Biosystems, California) and following the manufacturer's instructions.

Alternatively, antibodies of the invention can be produced by recombinant DNA techniques in a suitable expression system. The term "expression system" means a host cell and compatible vector under suitable conditions, e.g. for the expression of a protein coded for by foreign DNA carried by the vector and introduced to the host cell. Typically, a nucleic acid sequence encoding a monoclonal antibody of the invention, or a fragment thereof, may be included in any suitable expression vector which may then be introduced into suitable eukaryotic or prokaryotic hosts that will express the desired antibodies.

Accordingly, the invention further relates to a nucleic acid sequence encoding a monoclonal antibody of the invention, or a fragment thereof, and to a vector comprising such a nucleic acid sequence.

The terms "vector", "cloning vector" and "expression vector" mean the vehicle by which a DNA or RNA sequence encoding the antibody can be introduced into a host cell, so as to transform the host and promote expression (e.g. transcription and translation) of the introduced sequence. An expression vector is typically a plasmid, cosmid, episome, artificial chromosome, phage or a viral vector.

Such vectors generally comprise regulatory elements, such as a promoter, enhancer, terminator and the like, to cause or direct expression of said polypeptide upon administration to a subject. Examples of promoters and enhancers used in the expression vector for animal cell include early promoter and enhancer of SV40 (Mizukami T. et al. 1987), LTR promoter and enhancer of Moloney mouse leukemia virus (Kuwana Y et al. 1987), promoter (Mason JO et al. 1985) and enhancer (Gillies SD et al. 1983) of immunoglobulin H chain and the like.

Any expression vector for animal cell can be used, so long as a gene encoding the human antibody C region can be inserted and expressed. Examples of suitable vectors include pAGE107 (Miyaji H et al. 1990), pAGE103 (Mizukami T et al. 1987), pHSG274 (Brady G et al. 1984), pKCR (O'Hare K et al. 1981), pSG1 beta d2-4-(Miyaji H et al. 1990) and the like.

Other examples of vectors include replicating plasmids comprising an origin of replication, or integrative plasmids, such as for instance pUC, pcDNA, pBR, and the like.

Examples of viral vector include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO 94/19478.

Host cells are transfected, infected or transformed by a nucleic acid and/or an appropriate vector as above described. The term "transformation" means the introduction of a "foreign" (i.e. extrinsic or extracellular) gene, DNA or RNA sequence to a host cell, so that the host cell will express the introduced gene or sequence to produce a desired substance, typically a protein or enzyme coded by the introduced gene or sequence. Transformed host cells are encompassed within the scope of the invention.

Common expression systems include E. coli host cells and plasmid vectors, insect host cells and Baculovirus vectors, and mammalian host cells and vectors. Other examples of host cells include, without limitation, prokaryotic cells (such as bacteria) and eukaryotic cells (such as yeast cells, mammalian cells, insect cells, plant cells, etc.). Specific examples include E. coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from lymphoblasts, fibroblasts, embryonic cells, epithelial cells, nervous cells, adipocytes, etc.). Examples also include mouse SP2/0-Ag14 cell (ATCC CRL1581), mouse P3X63-Ag8.653 cell (ATCC CRL1580), CHO cell in which a dihydrofolate reductase gene (hereinafter referred to as "DHFR gene") is defective (Urlaub G et al; 1980), rat YB2/3HL.P2.G11.16Ag.20 cell (ATCC CRL1662, hereinafter referred to as "YB2/0 cell"), and the like. The YB2/0 cell is preferred, since ADCC activity of chimeric or humanized antibodies is enhanced when expressed in this cell.

Accordingly a method of producing a recombinant host cell expressing an antibody according to the invention, may comprise the steps consisting of : (i) introducing in vitro or ex vivo a recombinant nucleic acid or a vector as described above into a competent host cell, (ii) culturing in vitro or ex vivo the recombinant host cell obtained and (iii), optionally, selecting the cells which express and/or secrete said antibody or polypeptide.

Furthermore, a method of producing an antibody of the invention may comprise the steps consisting of: (i) culturing a transformed host cell as described above under conditions suitable to allow expression of said antibody; and (ii) recovering the expressed antibody.

Antibodies are suitably separated from the culture medium by conventional immunoglobulin purification procedures such as, for example, protein A-Sepharose, hydroxylapatite chromatography, gel electrophoresis, dialysis, or affinity chromatography.

The Fab of the present invention can be obtained by treating an antibody which specifically reacts with Tn antigen with a protease, papain. Also, the Fab can be produced by inserting DNA encoding Fab of the antibody into a vector for prokaryotic expression system, or for eukaryotic expression system, and introducing the vector into a procaryote or eucaryote (as appropriate) to express the Fab.

The F(ab')₂ of the present invention can be obtained treating an antibody which specifically reacts with Tn antigen with a protease, pepsin. Also, the F(ab')₂ can be produced by binding Fab' described below via a thioether bond or a disulfide bond.

The Fab' of the present invention can be obtained treating F(ab')₂ which specifically reacts with Tn antigen with a reducing agent, dithiothreitol. Also, the Fab' can be produced by inserting DNA encoding Fab' fragment of the antibody into an expression vector for prokaryote, or an expression vector for eukaryote, and introducing the vector into a prokaryote or eukaryote (as appropriate) to perform its expression.

The scFv of the present invention can be produced by obtaining cDNA encoding the VH and VL domains as previously described, constructing DNA encoding scFv, inserting the DNA into an expression vector for prokaryote, or an expression vector for eukaryote, and then introducing the expression vector into a prokaryote or eukaryote (as appropriate) to express the scFv. To generate a humanized scFv fragment, a well known technology called CDR grafting may be used, which involves selecting the complementary determining regions (CDRs) from a donor scFv fragment, and grafting them onto a human scFv fragment framework of known three dimensional structure (see, e. g., W098/45322; WO 87/02671; US5,859,205; US5,585,089; US4,816,567; EP0173494).

In particular, the invention further provides a nucleic acid comprising a sequence encoding an antibody, in particular a scFV, directed against Tn antigen which comprises, or consists of, (i) amino acids 1 to 244 of SEQ ID NO:24, or (ii) an amino acid sequence at least 85% identical to amino acid 1 to 244 of SEQ ID NO:24 provided said sequence comprises L-CDR1, L-CDR2, and L-CDR3 of the light chain shown in SEQ ID NO:8 and H-CDR1, H-CDR2, and H-CDR3 of the heavy chain shown in SEQ ID NO:6.

Preferably, said sequence encoding the scFV comprises nucleotides 1 to 732 of SEQ ID NO:23.

The invention also provides a nucleic acid comprising a sequence encoding 83D4scFv-hFc2 antibody. Thus the invention relates to a nucleic acid comprising a sequence encoding an antibody directed against Tn antigen which comprises, or consist of, (i) the amino acid sequence shown in SEQ ID NO:24, or (ii) an amino acid sequence at least 85%, preferably 90%, more preferably 95%, more preferably 97%, still preferably 99%, identical to SEQ ID NO:24 provided said amino acid sequence comprises L-CDR1, L-CDR2, and L-CDR3 of the light chain shown in SEQ ID NO:8 and H-CDR1, H-CDR2, and H-CDR3 of the heavy chain shown in SEQ ID NO:6.

Preferably, said sequence encoding 83D4scFv-hFc2 comprises SEQ ID NO:23.

It is also provided a vector comprising a nucleic acid comprising a sequence encoding the scFV as defined above. The invention also relates to a host cell comprising a nucleic acid or a vector as defined above.

### Immunoconjugates :

The invention further relates to an immunoconjugate comprising an antibody or fragment of the invention conjugated to an anti-cancer agent such as a cytotoxic agent or a growth inhibitory agent.

A "growth inhibitory agent" when used herein refers to a compound or composition which inhibits growth of a cell, especially ovarian cancer cell, either in *vitro* or *in vivo.* Examples of growth inhibitory agents include agents that block cell cycle progression, such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, and 5-fluorouracil. The taxanes (paclitaxel and docetaxel) are anticancer drugs both derived from the yew tree. Docetaxel, derived from the European yew, is a semisynthetic analogue of paclitaxel. Paclitaxel and docetaxel promote the assembly of microtubules from tubulin dimers and stabilize microtubules by preventing depolymerization, which results in the inhibition of mitosis in cells.

The term "cytotoxic agent" as used herein refers to a substance that inhibits or prevents the function of cells and/or causes destruction of cells. The term is intended to include radioactive isotopes (e.g. At²¹¹, I¹³¹, I¹²⁵, Y⁹⁰, Re¹⁸⁶, Re¹⁸⁸, Sm¹⁵³, Bi²¹², P³², and radioactive isotopes of Lu), chemotherapeutic agents, e.g., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, e.g., gelonin, ricin, saporin. A tumoricidal agent causes destruction of tumor cells.

Conjugation of the antibodies of the invention with cytotoxic agents or growth inhibitory agents may be made using a variety of bifunctional protein coupling agents including but not limited to N-succinimidyl (2-pyridyldithio) propionate (SPDP), succinimidyl (N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6diisocyanate), and bis-active fluorine compounds (such as 1,5-difluoro-2,4- dinitrobenzene). For example, a ricin immunotoxin can be prepared as described in Vitetta et al (Science. 1987 Nov 20;238(4830):1098-104). Carbon labeled 1-isothiocyanatobenzyl methyldiethylene triaminepentaacetic acid (MX-DTPA) is an exemplary chelating agent for conjugation of radionucleotide to the antibody (WO 94/11026).

The linker may be a "cleavable linker" facilitating release of the cytotoxic agent or growth inhibitory agent in the cell. For example, an acid-labile linker, peptidase-sensitive linker, photolabile linker, dimethyl linker or disulfide-containing linker (See e.g. U.S. Patent No. 5,208,020) may be used.

Alternatively, a fusion protein comprising the antibody and cytotoxic agent or growth inhibitory agent may be made, by recombinant techniques or peptide synthesis. The length of DNA may comprise respective regions encoding the two portions of the conjugate either adjacent one another or separated by a region encoding a linker peptide which does not destroy the desired properties of the conjugate.

The antibodies of the present invention may also be used in Antibody-Directed Enzyme Prodrug Therapy (ADEPT) by conjugating the antibody to a prodrug-activating enzyme which converts a prodrug (e.g. a peptidyl chemotherapeutic agent, see WO 81/01145) to an active anti-cancer drug (See, for example, WO 88/07378 and U.S. Patent No. 4,975,278). The enzyme component of the immunoconjugate useful for ADEPT includes any enzyme capable of acting on a prodrug in such a way so as to covert it into its more active, cytotoxic form. Enzymes that are useful in the method of this invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic fluorocytosine into the anticancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as O-galactosidase and neuraminidase useful for converting glycosylated prodrugs into free drugs; P-lactamase useful for converting drugs derivatized with P- lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. The enzymes can be covalently bound to the antibodies by techniques well known in the art such as the use of the heterobifunctional crosslinking reagents discussed above.

### Therapeutic methods and uses

Antibodies, fragments or immunoconjugates of the invention may be useful for treating cancer. The antibodies of the invention may be used alone or in combination with any suitable agent.

For instance the antibodies may be used in combination with molecules activating the cells of the immune system such as cytokines (e.g. interleukin 2, interleukin 15 or interferon gamma), ligands of toll-like receptors (e.g. CpG for Toll-like receptor 9, poly(I:C) for toll-like receptor 3, imidazoquinolinamines for toll-like receptors 7 and 8), antibodies activating immune cells (e.g. anti-CD40, anti-CD3), or recombinant proteins activating immune cells (e.g. Flt3-ligang, CD40-ligand).

Therapeutic monoclonal antibodies can lead to the depletion of cells bearing the antigen specifically recognized by the antibody. This depletion can be mediated through at least three mechanisms: antibody mediated cellular cytotoxicity (ADCC), complement dependent lysis, and direct anti-tumour inhibition of tumour growth through signals given via the antigen targeted by the antibody.

"Complement dependent cytotoxicity" or "CDC" refers to the lysis of a target cell in the presence of complement. Activation of the classical complement pathway is initiated by the binding of the first component of the complement system to antibodies which are bound to their cognate antigen. To assess complement activation, a CDC assay, e.g. as described in Gazzano-Santoro et al. (J Immunol Methods. 1997 Mar 28;202(2):163-71) may be performed.

"Antibody-dependent cell-mediated cytotoxicity" or "ADCC" refers to a form of cytotoxicity in which secreted antibodies bound onto Fc receptors (FcRs) present on certain cytotoxic cells (e.g. Natural Killer (NK) cells, neutrophils, and macrophages) enable these cytotoxic effector cells to bind specifically to an antigen-bearing target cell and subsequently kill the target cell. To assess ADCC activity of a molecule of interest, an *in vitro* ADCC assay, such as described in US Patent No. 5,500,362 or 5,821,337, may be performed.

In another embodiment antibodies of the invention may be conjugated to a growth inhibitory agent, cytotoxic agent, or a prodrug-activating enzyme as previously described. Antibodies of the invention may be indeed useful for targeting said growth inhibitory agent, cytotoxic agent, or a prodrug to the tumour cell expressing the Tn antigen.

Thus, an object of the invention relates to a method of treating cancer comprising administering a subject in need thereof with a therapeutically effective amount of an antibody, fragment or immunoconjugate of the invention.

Another object of the invention relates to the use of an antibody, fragment or immunoconjugate of the invention for the manufacture of a medicament intended for the treatment of cancer.

In the context of the invention, the term "treating" or "treatment", as used herein, means reversing, alleviating, inhibiting the progress of, or preventing the disorder or condition to which such term applies, or one or more symptoms of such disorder or condition. In particular, the treatment of cancer may consist in inhibiting the growth of cancer cells. Preferably such treatment also leads to the regression of tumor growth, i.e., the decrease in size of a measurable tumor. Most preferably, such treatment leads to the complete regression of the tumor.

According to the invention, the term "subject" or "subject in need thereof" is intended for a human or non-human mammal (such as a rodent (mouse, rat), a feline, a canine, or a primate) affected or likely to be affected with a cancer. Preferably, the subject is a human.

The term "therapeutically effective amount" is meant for a sufficient amount of antibody in order to treat said cancer, at a reasonable benefit/risk ratio applicable to any medical treatment. It will be understood, however, that the total daily usage of the antibodies and compositions of the present invention will be decided by the attending physician within the scope of sound medical judgment. The specific therapeutically effective dose level for any particular patient will depend upon a variety of factors including the disorder being treated and the severity of the disorder; activity of the specific antibody employed; the specific composition employed, the age, body weight, general health, sex and diet of the patient; the time of administration, route of administration, and rate of excretion of the specific antibody employed; the duration of the treatment; drugs used in combination or coincidental with the specific polypeptide employed; and like factors well known in the medical arts. For example, it is well known within the skill of the art to start doses of the compound at levels lower than those required to achieve the desired therapeutic effect and to gradually increase the dosage until the desired effect is achieved.

Antibodies of the invention may be used in combination with any other therapeutical strategy for treating cancer (e.g. external radiotherapy, chemotherapy or cytokines).

Preferably, the method according to the invention may be intended for the treatment of solid tumours, including both carcinoma and sarcoma, and lymphoma.

The Tn antigen is found exposed in about 90% of human carcinomas. Accordingly, the method of the invention may be operated for the treatment of any carcinoma, and in particular for the treatment of breast, ovarian, skin, head and neck, lung, colon, pancreas, prostate and bladder cancer.

Furthermore, the inventors have determined that the Tn antigen is exposed on Ewing's sarcoma cells. Thus, according to an embodiment, the invention also provides a treatment of Ewing's sarcoma.

The Tn antigen is also detected at the surface of Jurkat cells, which are a T ALLcell line. Hence, the method of treatment according to the invention may also be carried out for the treatment of Hodgkin lymphoma, non-Hodgkin lymphoma, or T ALL.

### Pharmaceutical compositions:

The nucleic acids, antibodies, fragments or conjugates of the invention may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The form of the pharmaceutical compositions, the route of administration, the dosage and the regimen naturally depend upon the condition to be treated, the severity of the illness, the age, weight, and sex of the patient, etc.

The pharmaceutical or therapeutic compositions of the invention can be formulated for a topical, oral, parenteral, intranasal, intravenous, intramuscular, subcutaneous or intraocular administration and the like.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

To prepare pharmaceutical compositions, an effective amount of the antibody may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

An antibody of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The antibodies of the invention may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds formulated for parenteral administration, such as intravenous or intramuscular injection, other pharmaceutically acceptable forms include, e.g. tablets or other solids for oral administration ; time release capsules ; and any other form currently used.

In certain embodiments, the use of liposomes and/or nanoparticles is contemplated for the introduction of antibodies into host cells. The formation and use of liposomes and/or nanoparticles are known to those of skill in the art.

Nanocapsules can generally entrap compounds in a stable and reproducible way. To avoid side effects due to intracellular polymeric overloading, such ultrafine particles (sized around 0.1 µm) are generally designed using polymers able to be degraded in vivo. Biodegradable polyalkyl-cyanoacrylate nanoparticles that meet these requirements are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 A, containing an aqueous solution in the core. The physical characteristics of liposomes depend on pH, ionic strength and the presence of divalent cations.

The invention will be further illustrated in view of the following Figures and Examples.

### FIGURES

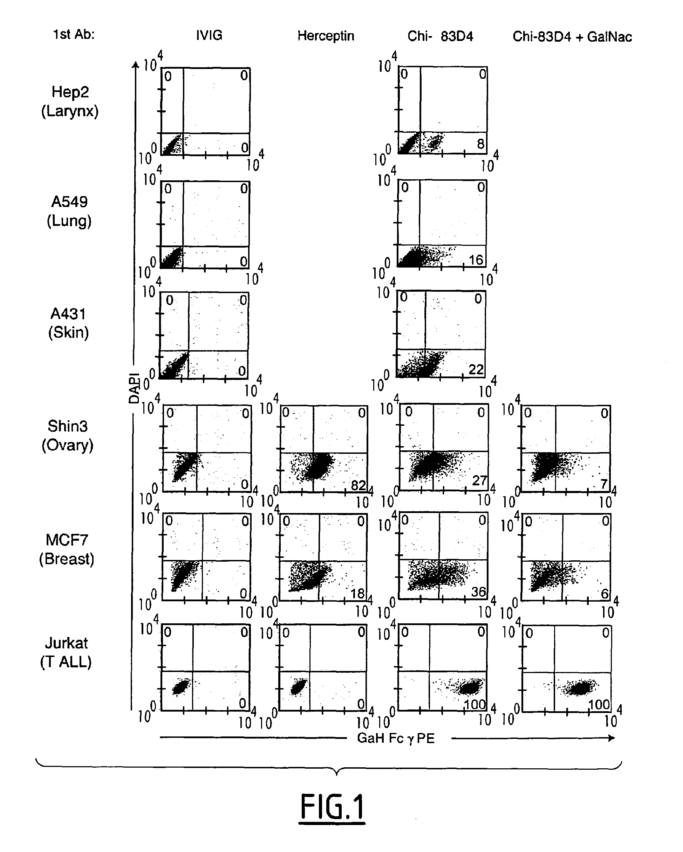

Figure 1 shows specific recognition of the Tn antigen by the chi-83D4 mAb at the surface of carcinoma tumor cell lines. Indicated tumor cells were labelled with the chi-83D4 mAb, or Herceptin, or IVIG (all at 20 µg/ml) and GaH Fc-γ PE. Tn specificity was determined by pre-incubating the chi-83D4 mAb with GalNac prior to cell labelling. Living cells (10,000) were then gated and analyzed for Tn or Her2 expression. Numbers in the quadrants indicated the percentages of cells.

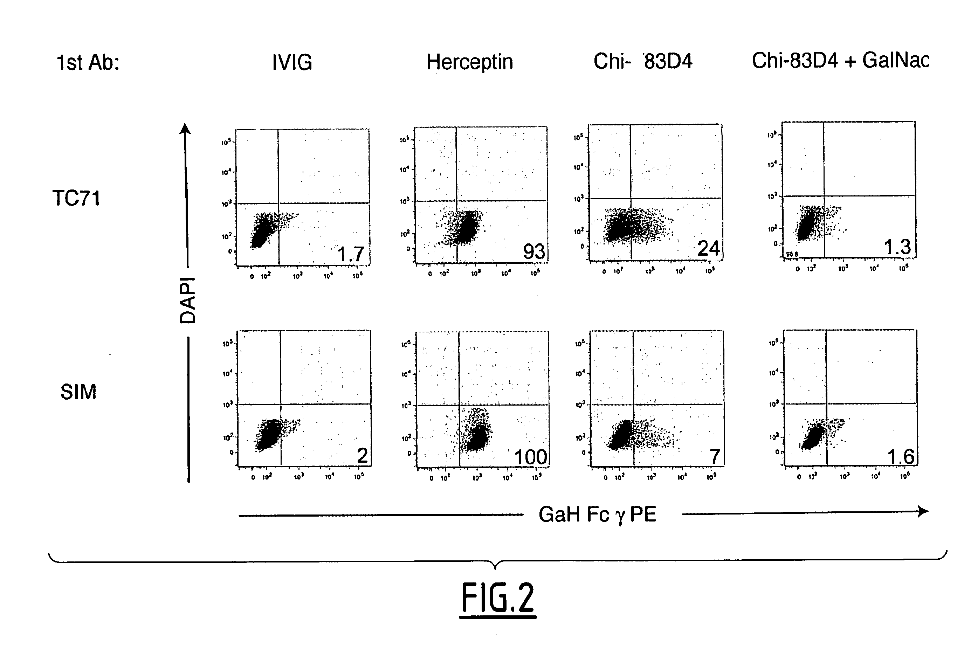

Figure 2 shows specific recognition of the Tn antigen by the chi-83D4 mAb at the surface of Ewing sarcoma cell lines. Two representative Ewing sarcoma cell lines were labeled with the chi-83D4 mAb, or Herceptin, or IVIG at 20 µg/ml and GaH Fc-γ PE. Tn specificity was assessed by pre-incubating the chi-83D4 mAb with GalNac prior to cell labeling. Living cells (10,000) were then gated and analyzed for Tn or Her expression. Numbers in the quadrants indicated the percentages of positive cells.

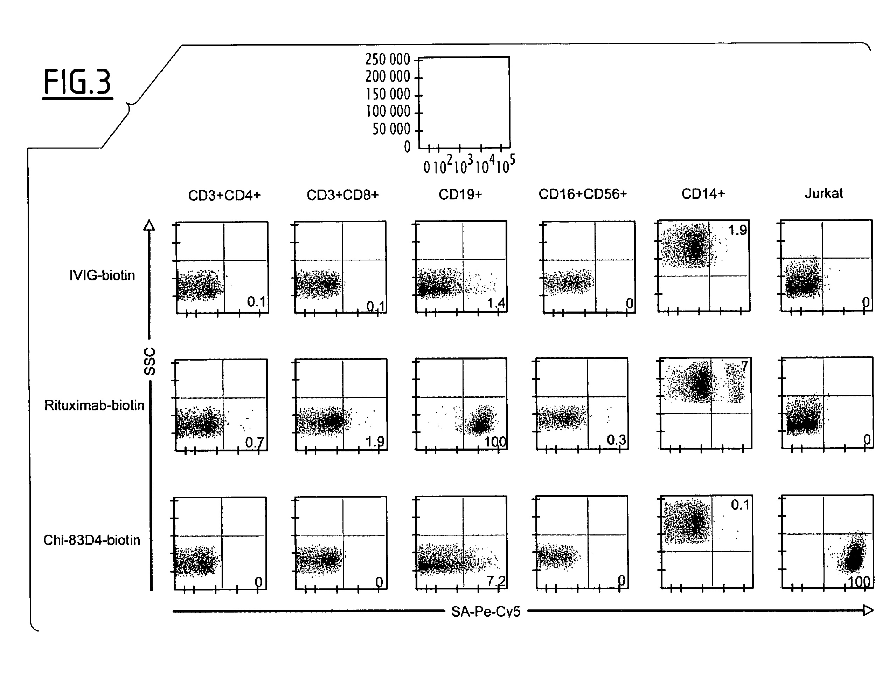

Figure 3 illustrates that the chi-83D4 mAb did not bind to normal human hematopoïetic cells. PBMC from normal healthy blood donors were first saturated with IVIG for 1 h, then labelled with the indicated combination of directly coupled mAbs to distinguish the different subpopulations. Cells were then labelled with the indicated biotinylated antibody (chi-83D4 or Rituximab or IVIG at 20 µg/ml) and SA-PECy5. Cells were counterstained with Dapi before acquisition. After gating living cells, PBMC subpopulations were analyzed for F13 positivity. Jurkat cells were labeled with each biotinylated antibody and SA-PC5. In each case, 10,000 cells were acquired. Percentages of cells are indicated. The X-axis is in logarithmic scale, and the Y-axis is in linear scale, as shown in the empty histogram.

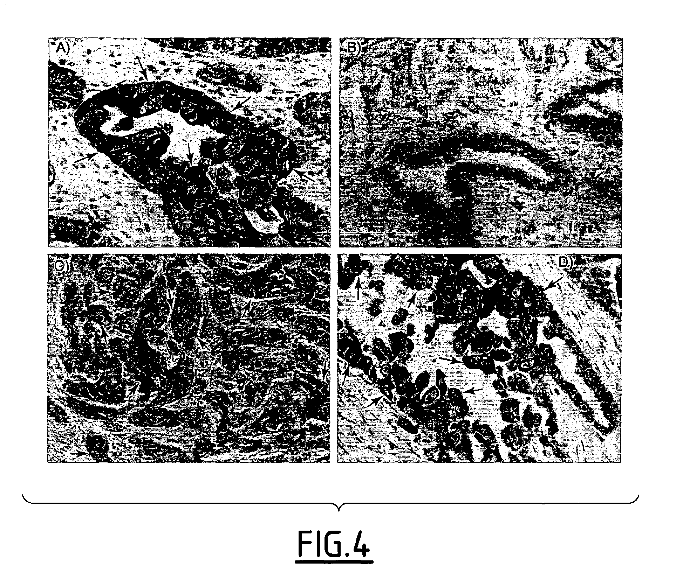

Figure 4 illustrates labeling of human tumors with the chi-83D4 mAb. A) Primary ovarian serous adenocarcinoma from patient 1. B) Normal ovary epithelium in patient 1. C) Primary ovarian serous adenocarcinoma from patient 2. D) Primary ovarian serous adenocarcinoma from patient 3. Anti-Tn-labeled tumor cells appear in dark. Arrows indicate examples of anti-Tn-labeled tumor cells.

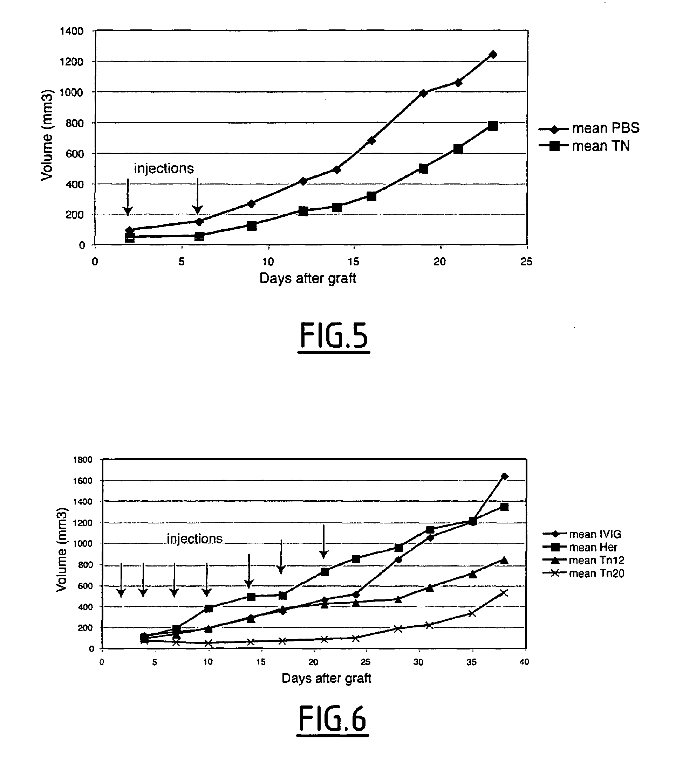

Figure 5 shows *in vivo* anti-tumor effect of the chi-83D4 mAb on a RAG/Hep2 model. RAG^{-/-} mice (n=5 in each group of mice) were grafted s.c. with Hep2 cells (5x10⁶) and injected i.p. at day 1 and day 6 with the chi-83D4 mAb (20 mg/kg/injection), or with the same volume of PBS. Tumor volumes were measured bi-weekly.

Figure 6 shows *in vivo* anti-tumor effect of the chi-83D4 mAb on a RAG/Shin3 model. RAG^{-/-} mice (n=5 for each group) were grafted s.c. with the Shin3 cell line (5x10⁶) and then injected i.p. every 3 days with the chi-83D4 mAb at 12 mg/kg/injection or at 20 mg/kg/injection (7 injections were performed). Control mice were treated with IVIG or Herceptin at 12 mg/kg/injection. Tumor volumes were measured bi-weekly.

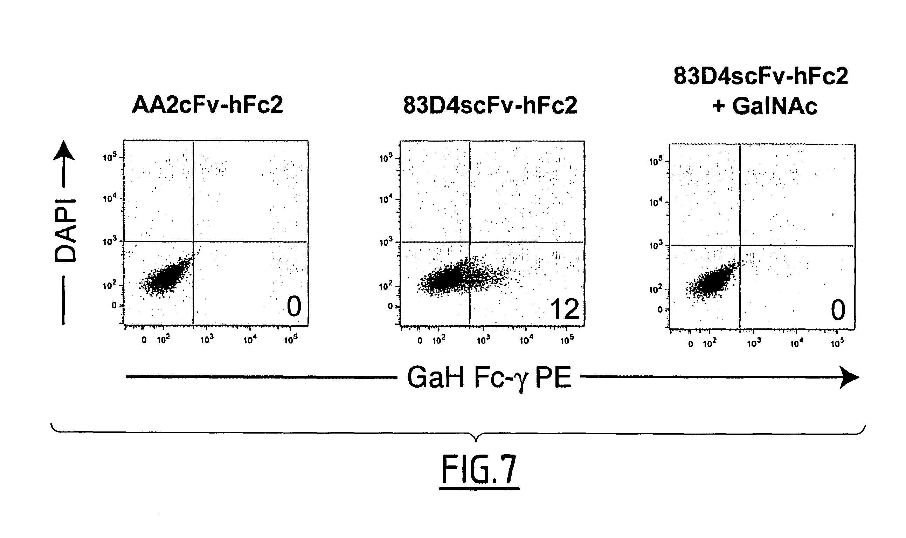

Figure 7 shows detection by FACS analysis of cell surface binding of 83D4scFv-hFc2. Jurkat cells were incubated with the control AA2scFv-hFc2-containing supernatants (Figure 7 left) or with 83D4scFv-hFc2-containing supernatants (Figures 7 center and right). Primary antibodies were detected by a secondary GaH Fc-γ PE. The specificity of the 83D4scFv-hFc2 labelling was assessed by pre-incubating the supernatant with GalNAc prior to cell labelling (Figure 7 right). Living cells (10,000) were then gated and analyzed for 83D4scFv-hFc-target expression. Numbers in the quadrants indicated the percentages of positive cells.

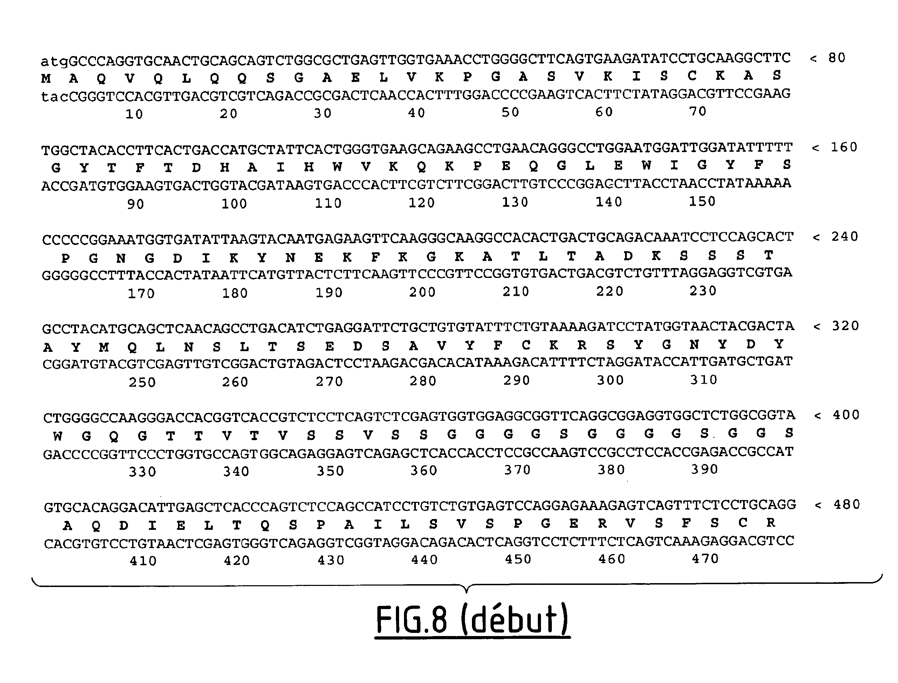

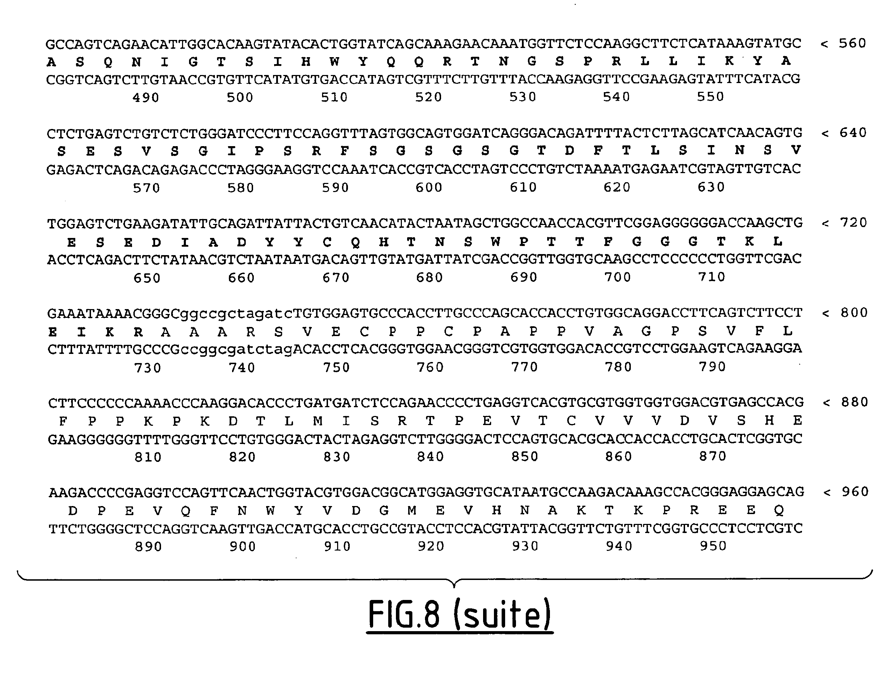

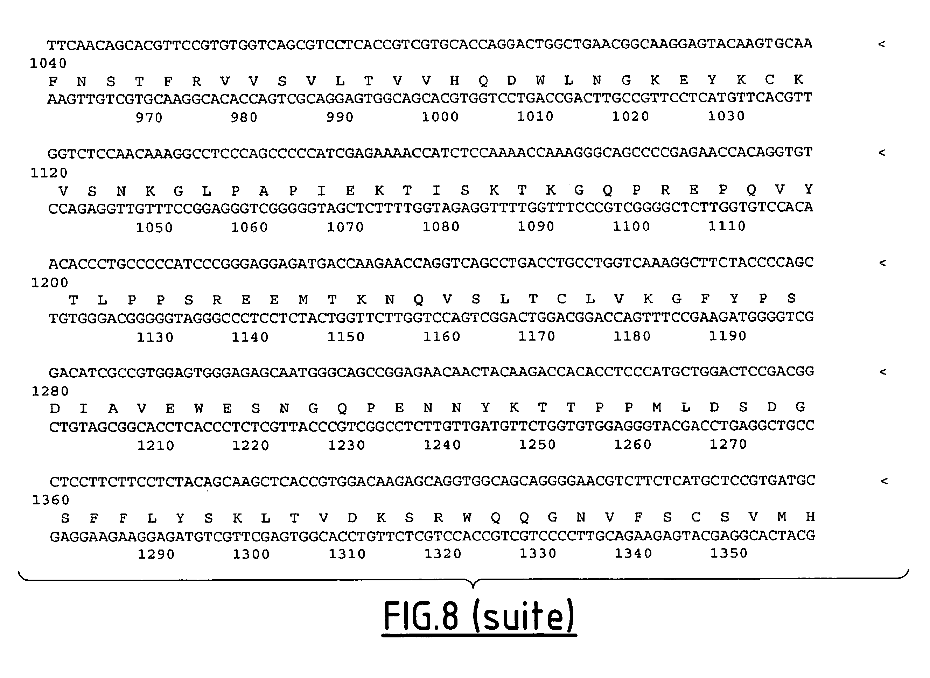

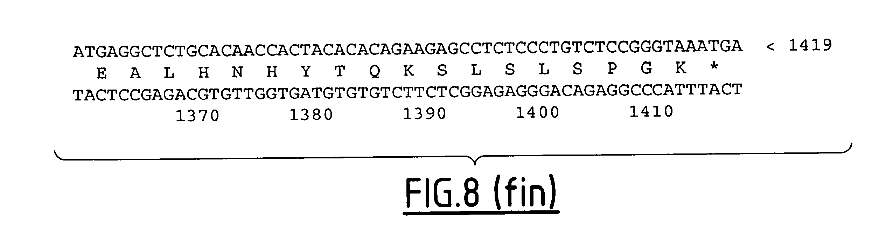

Figure 8 shows the sequence of 83D4scFv-hFc2 and scFv (in bold characters).

### EXAMPLES

### Example 1: Materials and methods

### 1. Cells

Hep2 (laryngeal head and neck epidermoïd carcinoma), MCF7 (breast carcinoma) and A549 (lung carcinoma) cell lines were kindly given by M. F. Poupon (Institut curie, Paris). Shin3 is an ovarian serous adenocarcinoma cell line (Imai, Oncology, 1990) and A431 is a skin epidermoïd carcinoma cell line provided by C. Cabella, Bioindustry Park del Canavese, Ivrea, Italy. Jurkat cells were given by A. Alcover (Institut Pasteur, Paris). The Ewing sarcoma cell lines (TC71 and SIM) were kindly provided by O. Delattre (Institut Curie, Paris).

PBMC were obtained from normal blood donors after purification on ficoll gradient.

### 2. Antibodies

The parental mouse anti-Tn mAb 83D4 (m83D4) has been previously described (Pancino, Hybridoma, 1990, 9:389-395). The chimeric anti-Tn (chi-83D4) mAb was constructed by fusion of the variable fragments of the parental m83D4 to the constant region of human IgG1 k (Oppezzo, Hybridoma, 2000, 19:229-239), and was produced and purified as explained below.

The chimeric anti-human CD20 mAb Rituximab^{®} was purchased from Hoffmann-La Roche Ltd, Basel, Switzerland. Intravenous immunoglobulin (Tegeline^{®}, IVIG) were obtained from the Laboratoire Français du Fractionnement et des Biotechnologies, Les Ulis, France. Chi-83D4 mAb, IVIG and Rituximab^{®} were biotinylated using NHS-LC-biotin (Pierce Biotechnology, Rockford, IL, U.S.A), following manufacturer's instructions. The humanized anti-human Her2 mAb trastuzumab (Herceptin^{®}) was obtained from Hoffmann-La Roche Ltd.

Directly conjugated mAbs CD3-FITC, CD16-FITC, CD19-FITC, CD4-PE, CD8-PE, and CD56-APC were purchased from Beckman Coulter immunotech, Marseille, France. CD14-APC was from Becton Dickinson, San Diego, CA, U.S.A.

### 3. Purification of the chi-83D4 mAb

X-63 cells transfected with vectors carrying VH and VL fragments of the m83D4 mAb fused to human Fc-γ1 and Fc-k fragments, respectively, were previously described (Oppezzo, Hybridoma, 2000). Cells were subcloned by limiting dilution in 96-well plates, and screened by flow cytometry for high antibody production. A subclone producing a high titer of the chi-83D4 antibody was selected and used throughout this work. These cells were grown in RPMI 1640 medium containing 10% of immunoglobulin-depleted foetal calf serum, and neomycin (0.2 mg/ml). Culture supernatants were then recovered, and the chi-83D4 mAb was purified on a protein A column, dialyzed against PBS, concentrated to 1 mg/ml and filtrated on 0.2 µm.

### 4. Sequencing of the chi-83D4 complete cDNA

The selected subclone (1 x 10⁶ cells) was lysed in RNABIe (Eurobio, Courtaboeuf, France), then total RNAs were extracted using phenol/chloroform, and reverse transcribed. cDNA was amplifed by polymerase chain reaction using the following primers. For the heavy chain, the VH-specific 5' primer: CAAACCATGGAATGGAGGTGGGTC (SEQ ID NO:1), and the CH3-specific 3' primer: TTTACCCGGAGACAGGGAGAGGCT (SEQ ID NO:2) were used. For the light chain, the VL-specific 5' primer: AGATGGTATCCACACCTCAGTTCC (SEQ ID NO:3), and the Ck-specific 3' primer: TCAACACTCTCCCCTGTTGAAGCTCTT (SEQ ID NO:4) were used. PCR reactions were performed using the Phusion DNA polymerase (Finnzymes Oy, Espoo, Finland) at 98°C 10 sec, 58°C 30 sec, 72°C 30 sec (35 cycles). PCR products were migrated on agarose gel, excised and purified using the nucleospin extract II kit (Macherey-Nagel, Düren, Germany). Sequences were performed using the Big dye Terminator V3.1 kit (Applied Biosystem, Warrington, UK).

### 5. FACS analysis

Tumor cells were stripped in PBS containing 0.5 M EDTA, washed in wash-buffer (PBS containing 0.5% BSA and 0.01 % sodium azide), and then incubated with the indicated antibodies at 20 µg/ml. After washing, cells were incubated with a Fab'₂ goat anti-serum specific for the Fc fragment of human IgG coupled to phyco-erythrin (GaH Fc-γ PE) (Jackson ImmunoResearch Laboratories, West Grove, PA, U.S.A). For inhibition experiments, the chi-83D4 mAb (20 µg/ml) was preincubated with synthetic GalNac (Sigma, Saint-Quentin Fallavier, France) at 0.1 M final concentration for 1 h on ice, before being used for cell labeling as above.

PBMC (1x10⁶ per sample) were first saturated with IVIG (50 mg/ml) for 1 h on ice, to avoid non-specific binding of antibodies to Fc receptors, before labeling with the indicated combination of directly conjugated-mAb. After washing, cells were then labeled with biotinylated-chi-83D4 mAb or -Rituximab^{®} or -IVIG at 20 µg/ml, and then incubated with streptavidin coupled to the phycoerythrin-cyanine 5 fluorochrome (SA-PE-Cy5). Cells were counterstained with Dapi (0.5 µg/ml, Molecular Probes) before acquisition on a LSRII cytofluorometer (Becton Dickinson) using the FACSDiva^{®} software, and analyzed using the FlowJo^{®} software (TreeStar Inc., Ashland, OR).

### 6. Immunohistochemistry

Tumors from 3 patients with primary ovarian serous adenocarcinoma, were analyzed. All patients were from the Institut Curie, Paris, France. Tissues sections from paraffin-embedded tumor samples were labeled with the biotinylated chi-83D4 mAb and avidin coupled to HRP.

### 7. Mouse xenograft models

Male and female RAG^{-/-} mice were provided by our animal facilities. Mice were injected s.c. in the right flank with 5x10⁵ tumor cells suspended in 100 µl matrigel (Becton Dickinson). The day after graft, mice were injected i.p as indicated with the chi-83D4 mAb or control antibodies (Herceptin or IVIG) or with the same volume of PBS. Tumor diameters were measured twice weekly using calipers. The tumor volume was estimated using the formula V= (length x width²)/2. Animals were monitored for overall activity, weight, and necropsy. Statistical analyses were performed using the Mann-Whitney test. This study was approved by the French Veterinary Department.

### Example 2: Sequencing of Chi-83D4

Total RNA were extracted from X63 cells transfected with vectors carrying VH and VL fragments of the m83D4 mAb fused to human Fc-γ1 and Fc-κ fragments respectively (Oppezzo, Hybridoma, 2000), reverse transcribed, amplified by PCR and sequenced as described in Materials and Methods. The complete sequence of chi-83D4 heavy and light chains are shown below.

chi-83D4 Heavy Chain complete cDNA sequence:
ATGGAATGGAGGTGGGTCTTTCTCTTCTTCCTGTCAGTAACTACAGG
TGTCCACTCCCAGGTTCAGCTGCAGCAGTCTGACGCTGAGTTGGTGAAAC
CTGGGGCTTCAGTGAAGATATCCTGCAAGGCTTCTGGCTACACCTTCACT
GACCATGCTATTCACTGGGTGAAGCAGAAGCCTGAACAGGGCCTGGAATG
GATTGGATATTTTTCCCCCGGAAATGGTGATATTAAGTACAATGAGAAGT
TCAAGGGCAAGGCCACACTGACTGCAGACAAATCCTCCAGCACTGCCTAC
ATGCAGCTCAACAGCCTGACATCTGAGGATTCTGCTGTGTATTTCTGTAA
AAGATCCTATGGTAACTACGACTACTGGGGCCAAGGCACCACTCTCACAG
TCTCCTCAGCCTCCACCAAGGGCCCATCGGTCTTCCCCCTGGCACCCTCC
TCCAAGAGCACCTCTGGGGGCACAGCGGCCCTGGGCTGCCTGGTCAAGGA
CTACTTCCCCGAACCGGTGACGGTGTCGTGGAACTCAGGCGCCCTGACCA
GCGGCGTGCACACCTTCCCGGCTGTCCTACAGTCCTCAGGACTCTACTCC
CTCAGCAGCGTGGTGACCGTGCCCTCCAGCAGCTTGGGCACCCAGACCTA
CATCTGCAACGTGAATCACAAGCCCAGCAACACCAAGGTGGACAAGAAAG
TTGAGCCCAAATCTTGTGACAAAACTCACACATGCCCACCGTGCCCAGCA
CCTGAACTCCTGGGGGGACCGTCAGTCTTCCTCTTCCCCCCAAAACCCAA

GGACACCCTCATGATCTCCCGGACCCCTGAGGTCACATGCGTGGTGGTGG
ACGTGAGCCACGAAGACCCTGAGGTCAAGTTCAACTGGTACGTGGACGGC
GTGGAGGTGCATAATGCCAAGACAAAGCCGCGGGAGGAGCAGTACAACAG
CACGTACCGTGTGGTCAGCGTCCTCACCGTCCTGCACCAGGACTGGCTGA
ATGGCAAGGAGTACAAGTGCAAGGTCTCCAACAAAGCCCTCCCAGCCCCC
ATCGAGAAAACCATCTCCAAAGCCAAAGGGCAGCCCCGAGAACCACAGGT
GTACACCCTGCCCCCATCCCGGGATGAGCTGACCAAGAACCAGGTCAGCC
TGACCTGCCTGGTCAAAGGCTTCTATCCCAGCGACATCGCCGTGGAGTGG
GAGAGCAATGGGCAGCCGGAGAACAACTACAAGACCACGCCTCCCGTGCT
GGACTCCGACGGCTCCTTCTTCCTCTACAGCAAGCTCACCGTGGACAAGA
GCAGGTGGCAGCAGGGGAACGTCTTCTCATGCTCCGTGATGCATGAGGCT
CTGCACAACCACTACACGCAGAAGAGCCTCTCCCTGTCTCCGGGTAAA
(SEQ ID NO:5).

chi-83D4 Heavy Chain :
MEWRWVFLFFLSVTTGVHSQVQLQQSDAELVKPGASVKISCKASGYTFT
DHAIHWVKQKPEQGLEWIGYFSPGNGDIKYNEKFKGKATLTADKSSSTAY
MQLNSLTSEDSAVYFCKRSYGNYDYWGQGTTLTVSSASTKGPSVFPLAPS
SKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPA
PELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEW
ESNGQPENNYKTTPPVLDSDGSFFLYSKLTVDKSRWQQGNVFSCSVMHEA
LHNHYTQKSLSLSPGK
(SEQ ID NO:6).

The 135 N-terminal amino acids (bold characters) of SEQ ID NO:6 comprise of the VH region. The Chi-83D4 V_{H} domain sequenced by the Inventors differs from the VH region of mouse 83D4 anti-Tn antibody deposited in GenPept under accession number AAG02617 by a substitution in position 4 (R4 in Chi-83D4 VH, and S4 in 83D4 VH).

The VH region (bp 1-405, amino-acids 1-135) included the CDR1 (bp 148-162, amino-acids DHAIH, SEQ ID NO:28), the CDR2 (bp 205-255, amino-acids YFSPGNGDIKYNEKFKG, SEQ ID NO:29) and the CDR3 (bp 352-372, amino-acids SYGNYDY, SEQ ID NO:30) fragments. The Fc region (bp 406-1395, amino-acids 136-465) included the CH1 (bp 406-699, amino-acids 136-233), the CH2 (bp 745-1074, amino-acids 249-358) and the CH3 (bp 1075-1395, amino-acids 359-465) fragments. A sequence of 45 bp (bp 700-744, amino-acids 234-248) is inserted between the CH1 and the CH2 fragments.

chi-83D4 Light Chain complete cDNA sequence:
ATGGTATCCACACCTCAGTTCCTTGTATTTTTGCTTTTCTGGATTCCAGC
CTCCAGAGGTGACATCTTGCTGACTCAGTCTCCAGCCATCCTGTCTGTGA
GTCCAGGAGAAAGAGTCAGTTTCTCCTGCAGGGCCAGTCAGAACATTGGC
ACAAGTATACACTGGTATCAGCAAAGAACAAATGGTTCTCCAAGGCTTCT
CATAAAGTATGCTTCTGAGTCTGTCTCTGGGATCCCTTCCAGGTTTAGTG
GCAGTGGATCAGGGACAGATTTTACTCTTAGCATCAACAGTGTGGAGTCT
GAAGATATTGCAGATTATTACTGTCAACATACTAATAGCTGGCCAACCAC
GTTCGGAGGGGGGACCAAACTGGAAATAAAACGAACTGTGGCTGCACCAT
CTGTCTTCATCTTCCCGCCATCTGATGAGCAGTTGAAATCTGGAACTGCC
TCTGTTGTGTGCCTGCTGAATAACTTCTATCCCAGAGAGGCCAAAGTACA
GTGGAAGGTGGATAACGCCCTCCAATCGGGTAACTCCCAGGAGAGTGTCA
CAGAGCAGGACAGCAAGGACAGCACCTACAGCCTCAGCAGCACCCTGACG
CTGAGCAAAGCAGACTACGAGAAACACAAAGTCTACGCCTGCGAAGTCAC
CCATCAGGGCCTGAGCTCGCCCGTCACAAAGAGCTTCAACAGGGGAGAGT
GT
(SEQ ID NO:7).

chi-83D4 Light Chain:
MVSTPQFLVFLLFWIPASRGDILLTQSPAILSVSPGERVSFSCRASQNIG
TSIHWYQQRTNGSPRLLIKYASESVSGIPSRFSGSGSGTDFTLSINSVES
EDIADYYCQHTNSWPTTFGGGTKLEIKRTVAAPSVFIFPPSDEQLKSGTA
SVVCLLNNFYPREAKVQWKVDNALQSGNSQESVTEQDSKDSTYSLSSTLT
LSKADYEKHKVYACEVTHQGLSSPVTKSFNRGEC
(SEQ ID NO:8).

This sequence is 100% identical over the 127 N-terminal amino acids (bold characters) with the VL region of mouse 83D4 anti-Tn antibody deposited in GenPept under accession number AAG02616.

The VL region (bp 1-381, amino-acids 1-127) included the CDR1 (bp 130-162, amino-acids RASQNIGTSIH, SEQ ID NO:25), the CDR2 (bp 208-228, amino-acids YASESVS, SEQ ID NO:26) and the CDR3 (bp 325-351, amino-acids QHTNSWPTT, SEQ ID NO:27) fragments. The C kappa region comprised the bp 382-702, amino-acids 128-234.

### Example 3: Specific cell surface recognition of Tn antigen by the chi-83D4 mAb in a panel of human epithelial tumor cell lines

Expression of the Tn antigen has been reported in several types of epithelial tumors by immunohistochemistry (Springer, Science, 1984; 224(4654):1198-206). To verify whether the chi-83D4 mAb could be used in tumor immunotherapy, it was first checked whether it could detect the Tn antigen at the plasma membrane of several carcinoma cell lines by flow cytometry.

As shown in Figure 1, the chi-83D4 mAb could label various types of carcinoma cell lines from larynx, lung, skin, ovary, breast, as well as Jurkat T ALL. All carcinoma cell lines expressed some levels of Her2, but no non-specific labeling was seen with IVIG used as control human antibodies. The chi-83D4 labeling was specific for the Tn antigen since it could be inhibited by synthetic GalNac. Thus, these epithelial tumor cell lines express the Tn antigen at the plasma membrane, which could be specifically targeted by our chi-83D4 mAb.

In addition, it was found that the chi-83D4 mAb could bind to the plasma membrane of two Ewing sarcoma cell lines, and that this binding was specific for the Tn antigen (Figure 2). This result thus demonstrates for the first time the expression of the Tn antigen in Ewing sarcoma cells.

### Example 4: The chi-83D4 mAb did not bind to normal mature human hematopoietic cells

Ideal therapeutic mAbs should be specific for tumor cells and should not recognize normal cells. Thus, the reactivity of the chi-83D4 mAb with normal mature human hematopoïetic cells was analyzed. For this purpose, subpopulations of PBMC from healthy blood donors were analyzed for biotinylated chi-83D4 mAb or control IVIG or Rituximab^{®} labeling. As shown in Figure 3, only a small fraction (7%) of B cells bound the biotinylated chi-83D4 mAb, whereas CD4+ and CD8+ T cells, NK cells or monocytes did not. The biotinylated normal human immunoglobulin IVIG did not bind to any subpopulation, whereas biotinylated Rituximab was still able to label B cells, and biotinylated chi-83D4 mAb labeled Jurkat cells efficiently.

### Example 5: The chi-83D4 mAb specifically labeled human epithelial tumors

It was next determined whether the chi-83D4 mAb could detect the Tn antigen in patients' primary tumors ex-vivo (Figure 4). Tissue sections were labelled with the chi-83D4 mAb and avidin-HRP.

Representative data of 3 primary ovarian serous adenocarcinomas are shown in Figure 4. All 3 ovarian cancers expressed Tn significantly, with 70-80% of tumor cells being moderately to strongly labeled. The labeling was located in the cytosol or at the plasma membrane as shown for patient 1 (Figure 4 A) and patient 3 (Figure 4 D). It was also seen in the para-nuclear region for patient 2 (Figure 4 C). Neither the normal ovarian epithelium nor the Fallopian tube epithelium was labeled with the chi-83D4 mAb (Figure 4 B). Thus, the chi-83D4 mAb could specifically label the human ovarian carcinomas.

### Example 6: Inhibition of tumor growth after treatment with the chi-83D4 mAb in mouse xenograft models

Finally, it was investigated whether the chi-83D4 mAb could affect human tumor growth in immunodeficient mice.

As shown in Figure 5, RAG^{-/-} mice grafted with the laryngeal Hep2 cell line and treated with the chi-83D4 mAb (20 mg/kg/injection) displayed a significant reduced tumor growth as compared to control mice injected with PBS (Mann Whitney test: p<0.05 until day 16).

Moreover, the effect of two different doses of the chi-83D4 mAb was assessed on the growth of the ovarian cell line Shin3 grafted in RAG^{-/-} mice (Figure 6). In mice treated with the chi-83D4 mAb at 20 mg/kg/injection, the tumor growth was significantly delayed as compared to mice treated with IVIG or Herceptin (Mann Whitney test: p<0.05 from d7 to d38 and from d7 to d31 respectively). A weaker reduction in Shin3 tumor growth was observed when mice were treated with a lower dose of the chi-83D4 mAb (12 mg/kg/injection, Mann Whitney test: p<0.05 from d28 to d38 for IVIG and at d31 for Herceptin).

No sign of overall toxicity was observed in each of these experiments (no loss of weigh, abnormal activity or macroscopic organ lesion), as well as in tumor-free mice injected with the chi-83D4 mAb alone.

Thus, the chi-83D4 mAb was able to significantly reduce the growth of these two human carcinomas without any apparent sign of toxicity, suggesting that it could be a good candidate for human anti-cancer immunotherapy.

### Example 7: Obtention of a single chain Fv (scFv) anti-Tn derived from the hybidoma 83D4 cell line.

### mRNA isolation

Total mRNA was isolated from 6.10⁶ hybridoma cells with the GenElute^{™} Mammalian Total RNA Miniprep Kit (Sigma).

### cDNA synthesis

cDNA was obtained by reverse transcription of total RNA with First Strand cDNA Synthesis Kit (Fermentas) by priming 5 µg of RNA with oligo(dT) primers.

### PCR in two steps

Cloning of rearranged V_{H} and V_{L} genes was carried out by two PCR amplifications with Pfu DNA Polymerase (Promega).

The first PCR was performed with VHBack and VHFor (a mixture of 32 degenerated oligonucleotides optimized for amplification of murine V_{H} cDNA) and with VKBack and VKFor (mix of 4 primers) for amplification of V_{K} cDNA.
**VH Back** AG GT**S MAR** CTG CAG **S**AG TC**W** GG (SEQ ID NO:9)
VH For TGA GGA GAC GGT GAC CGT GGT CCC TTG GCC CC (SEQ ID NO:10)
**VK Back** GAC ATT GAG CTC ACC CAG TCT CCA (SEQ ID NO:11)
**VKFor (equimolar mix of the 4 primers)**
MJK1FONX CCG TTT GAT TTC CAG CTT GGT GCC (SEQ ID NO:12)
MJK2FONX CCG TTT TAT TTC CAG CTT GGT CCC (SEQ ID NO:13)
MJK3FONX CCG TTT TAT TTC CAA CTT TGT CCC (SEQ ID NO:14)
MJK4FONX CCG TTT CAG CTC CAG CTT GGT CCC (SEQ ID NO:15)
S= C or G; M=A or C; R= A or G; W= A or T;

Purified PCR products were submitted to a second PCR amplification with primers adding restrictions sites at the 5' and 3' ends (SfiVHBack and XhoVHFor for the VH ; ApaLIVKBack and NotVKFor for the VK).
**Sf*i* VH Back TA** CTC GCG GCC CAG CCG GCC ATG GCC CAG GT**S MAR** CTG CAG **S**AG TC (SEQ ID NO:16)
***Xho* VHFor** CC GCT CGA GAC TGA GGA GAC GGT GAC CGT (SEQ ID NO:17)
***ApaLl VK* Back** ACCGCCTCCACCAGT GCACAG GAC ATT GAG CTC ACC CAG (SEQ ID NO: 18)

### NotVKFor

TTTTCCTTTTGCGGCCGC CCG TTT GAT TTC CAG CTT GGT GCC (SEQ ID NO:19)
TTTTCCTTTTGCGGCCGC CCG TTT TAT TTC CAG CTT GGT CCC (SEQ ID NO:20)
TTTTCCTTTTGCGGCCGC CCG TTT TAT TTC CAA CTT TGT CCC (SEQ ID NO:21)
TTTTCCTTTTGCGGCCGC CCG TTT CAG CTC CAG CTT GGT CCC (SEQ ID NO:22)

### Cloning into a phagemide expression vector

PCR products were cloned by digestion and ligation in two steps in the pHEN2 vector containing a synthetic flexible linker between the VH and the VK.

*Subcloning into a mammalian vector comprising the Hinge+CH2+CH3 from human IgG2 Fc*

The subcloning has been performed by digestion and ligation into the modified pFuse-hFc2(IL2ss) vector (Invivogen). The pFuse vector has been mutagenized to insert cloning sites for scFv cloning (Ncol and Notl). This vector contains the Fc region of the human IgG2 which comprises the CH2 and CH3 domains of the heavy chain and the Hinge region.

The cDNA and amino acid sequences of 83D4scFv-hFc2 are shown on Figure 8 and in SEQ ID NO:23 and SEQ ID NO:24, respectively.

83D4scFv is shown in bold on Figure 8 (nucleotides 1 to 732 of SEQ ID NO:23 and amino acids 1 to 244 of SEQ ID NO:24).

### Secretion of recombinant 83D4scFv-hFc2 by transfected CHO cells.

CHO cells were transfected to transiently express the hF2c-fused 83D4scFv recombinant antibody (h83D4) or the hF2c-fused scFv-AA2 antibody (hAA2) used as a control. Culture supernatants were collected 3 days after transfection and 30 µl were analyzed by western blotting to reveal the secreted 50kD recombinant antibodies.

### Validation of 83D4scFv-hFc2 cell surface binding and specificity

The recognition of the Tn antigen at the cell surface by the 83D4scFv-hFc2 was assessed by flow cytometry. Jurkat cells (5 x 10⁵/sample) were washed in wash buffer (PBS containing 0.5% BSA and 0.01 % sodium azide) and incubated with pure supernatant of CHO cells transiently transfected with the 83D4scFv-hFc2 construct or with the control AA2scFv-hFc2 (Anti-Rab6•GTP scFv fused with hFc) construct for 15 min on ice. After washing, cells were incubated with a Fab'2 goat anti-serum specific for the Fc fragment of human IgG coupled to phyco-erythrin (GaH Fc-γ PE) (Jackson ImmunoResearch Laboratories, West Grove, PA, U.S.A). The specificity of the labelling was determined by inhibition experiments. The 83D4scFv-hFc2-containing supernatant was preincubated with synthetic GalNAc (Sigma, Saint-Quentin Fallavier, France) at 0.1 M final concentration for 1 h on ice, before being used for cell labelling as above. Cells were acquired on a LSRII cytofluorometer (Becton Dickinson) using the FACSDiva^{®} software, and analyzed using the FlowJo^{®} software.

The 83D4scFv-hFc2 was found to bind specifically to Jurkat cells by flow cytometry (Figure 7). As shown in the Figures, a significant shift in the F12 fluorescence was observed in cells incubated with the 83D4scFv-hFc2 construct, but not in control cells. Moreover, this shift was completely inhibited when the 83D4scFv-hFc2-containing-supernatant was pre-incubated with the antigen GalNac. Thus, this experiment indicates that the recombinant 83D4scFv-hFc2 behaves like the 83D4 antibody and specifically detects the Tn antigen present at the surface of Jurkat cells.

## Claims

1. A pharmaceutical composition comprising an antibody directed against Tn antigen comprising a variable light (VL) chain which comprises L-CDR1 (SEQ ID NO:25), L-CDR2 (SEQ ID NO:26), and L-CDR3 (SEQ ID NO:27) and a variable heavy (VH) chain which comprises H-CDR1 (SEQ ID NO:28), H-CDR2 (SEQ ID NO:29), and H-CDR3 (SEQ ID NO:30), together with a pharmaceutically acceptable carrier.

2. The pharmaceutical composition according to claim 1, wherein said antibody comprises (i) amino acids 1 to 127 of SEQ ID NO:8 or an amino acid sequence at least 85% identical to amino acids 1 to 127 of SEQ ID NO:8, and (ii) amino acids 1 to 135 of SEQ ID NO:6 or an amino acid sequence at least 85% identical to amino 1 to 135 of SEQ ID NO:6.

3. The pharmaceutical composition according to claim 1 or 2, wherein said antibody comprises (i) the light chain shown in SEQ ID NO:8 or an amino acid sequence at least 85% identical to SEQ ID NO:8 and (ii) the heavy chain shown in SEQ ID NO:6 or an amino acid sequence at least 85% identical to SEQ ID NO:6.

4. The pharmaceutical composition according to claim 1 or 2, wherein said antibody further comprises human constant regions of heavy (CH) and light chains (CL).

5. The pharmaceutical composition according to claim 1, wherein said L-CDR1, L-CDR2, and L-CDR3 of the light chain and said H-CDR1, H-CDR2, and H-CDR3 of the heavy chain are inserted into human framework regions.

6. The pharmaceutical composition according to claim 5, wherein said antibody further comprises human constant regions of heavy (CH) and light chains (CL).

7. The pharmaceutical composition according to any one of claims 1 to 6, wherein said antibody directed against Tn antigen is an antibody fragment.

8. The pharmaceutical composition according to any one of claims 1 to claim 7, wherein said antibody directed against Tn antigen comprises amino acids 1 to 244 of SEQ ID NO:24, or an amino acid sequence at least 85% identical to amino acid 1 to 244 of SEQ ID NO:24.

9. The pharmaceutical composition according to any one of claims 1 to claim 8, wherein said antibody directed against Tn antigen comprises the amino acid sequence of SEQ ID NO:24, or an amino acid sequence at least 85% identical to the amino acid sequence of SEQ ID NO:24.

10. An immunoconjugate comprising an antibody as defined in any one of claims 1 to 9, conjugated to an anti-cancer agent.

11. The immunoconjugate according to claim 10, wherein said anti-cancer agent is a growth inhibitory agent selected from the group consisting of vincas, taxanes, topoisomerase II inhibitors, DNA alkylating agents, taxanes, docetaxel, and paclitaxel; or a cytotoxic agent selected from the group consisting of radioactive isotopes, chemotherapeutic agents, enzymes, antibiotics, and toxins.

12. A pharmaceutical composition comprising an immunoconjugate according to claim 10 or 11, together with a pharmaceutically acceptable carrier.

13. Use of a monoclonal antibody as defined in any one of claims 1 to 9, or of an immunoconjugate according to claim 10 or 11, for the manufacture of a medicament intended for the treatment of cancer.

14. The use according to claim 13, wherein said medicament is intended for the treatment of solid tumours and lymphoma.

15. The use according to claim 13 or 14, wherein said medicament is intended for the treatment of breast, ovarian, skin, larynx, lung, colon, prostate or bladder cancer, Ewing's sarcoma, Hodgkin lymphoma or non-Hodgkin lymphoma.

16. An antibody directed against Tn antigen which comprises (i) amino acids 1 to 244 of SEQ ID NO:24, or (ii) an amino acid sequence at least 95% identical to amino acid 1 to 244 of SEQ ID NO:24 provided said sequence comprises L-CDR1 (SEQ ID NO:25), L-CDR2 (SEQ ID NO:26), and L-CDR3 (SEQ ID NO:27) and H-CDR1 (SEQ ID NO:28), H-CDR2 (SEQ ID NO:29), and H-CDR3 (SEQ ID NO:30).

17. An antibody according to claim 16, which comprises (i) the amino acid sequence of SEQ ID NO:24, or (ii) an amino acid sequence at least 85% identical to SEQ ID NO:24.

18. A nucleic acid comprising a sequence encoding the antibody according to claim 16 or 17.

19. The nucleic acid according to claim 18, wherein said sequence comprises nucleotides 1 to 732 of SEQ ID NO:23.

20. The nucleic acid according to claim 18 or 19, wherein said sequence comprises SEQ ID NO:23.

21. A vector comprising a nucleic acid according to any one of claims 18 to 20.

22. A host cell comprising a nucleic acid according to any one of claims 18 to 20 or a vector according to claim 21.

23. A method of producing an antibody according to claim 16 or 17 which comprises the steps consisting of: (i) culturing a host cell according to claim 22; and (ii) recovering the expressed antibody.


SEQUENCE LISTING

<110>  Institut Curie
       INSERM

<120>  An antibody specific for the Tn antigen for the treatment of
       cancer

<130>  BET 07P0207

<160>  30

<170>  PatentIn version 3.4

<210>  1
<211>  24
<212>  DNA
<213>  Artificial

<220>
<223>  primer

<400>  1
caaaccatgg aatggaggtg ggtc                                              24


<210>  2
<211>  24
<212>  DNA
<213>  Artificial

<220>
<223>  primer

<400>  2
tttacccgga gacagggaga ggct                                              24


<210>  3
<211>  24
<212>  DNA
<213>  Artificial

<220>
<223>  primer

<400>  3
agatggtatc cacacctcag ttcc                                              24


<210>  4
<211>  27
<212>  DNA
<213>  Artificial

<220>
<223>  primer

<400>  4
tcaacactct cccctgttga agctctt                                           27

<210> 5
<211> 1395
<212> DNA
<213> Artificial

<220>
<223> Chi-83D4 Heavy Chain cDNA


<220>
<221> misc_feature
<222> (1)..(405)
<223> VH region

<220>
<221> misc_feature
<222> (148)..(162)
<223> H-CDR1

<220>
<221> misc_feature
<222> (205)..(255)
<223> H-CDR2

<220>
<221> misc_feature
<222> (352)..(372)
<223> H-CDR3

<400> 5
atggaatgga ggtgggtctt tctcttcttc ctgtcagtaa ctacaggtgt ccactcccag 60

gttcagctgc agcagtctga cgctgagttg gtgaaacctg gggcttcagt gaagatatcc 120

tgcaaggctt ctggctacac cttcactgac catgctattc actgggtgaa gcagaagcct 180

gaacagggcc tggaatggat tggatatttt tcccccggaa atggtgatat taagtacaat 240

gagaagttca agggcaaggc cacactgact gcagacaaat cctccagcac tgcctacatg 300

cagctcaaca gcctgacatc tgaggattct gctgtgtatt tctgtaaaag atcctatggt 360

aactacgact actggggcca aggcaccact ctcacagtct cctcagcctc caccaagggc 420

ccatcggtct tccccctggc accctcctcc aagagcacct ctgggggcac agcggccctg 480

ggctgcctgg tcaaggacta cttccccgaa ccggtgacgg tgtcgtggaa ctcaggcgcc 540

ctgaccagcg gcgtgcacac cttcccggct gtcctacagt cctcaggact ctactccctc 600

agcagcgtgg tgaccgtgcc ctccagcagc ttgggcaccc agacctacat ctgcaacgtg 660

aatcacaagc ccagcaacac caaggtggac aagaaagttg agcccaaatc ttgtgacaaa 720

actcacacat gcccaccgtg cccagcacct gaactcctgg ggggaccgtc agtcttcctc 780

ttccccccaa aacccaagga caccctcatg atctcccgga cccctgaggt cacatgcgtg 840

gtggtggacg tgagccacga agaccctgag gtcaagttca actggtacgt ggacggcgtg 900

gaggtgcata atgccaagac aaagccgcgg gaggagcagt acaacagcac gtaccgtgtg 960

gtcagcgtcc tcaccgtcct gcaccaggac tggctgaatg gcaaggagta caagtgcaag 1020

gtctccaaca aagccctccc agcccccatc gagaaaacca tctccaaagc caaagggcag 1080

ccccgagaac cacaggtgta caccctgccc ccatcccggg atgagctgac caagaaccag 1140

gtcagcctga cctgcctggt caaaggcttc tatcccagcg acatcgccgt ggagtgggag 1200

agcaatgggc agccggagaa caactacaag accacgcctc ccgtgctgga ctccgacggc 1260

tccttcttcc tctacagcaa gctcaccgtg gacaagagca ggtggcagca ggggaacgtc 1320

ttctcatgct ccgtgatgca tgaggctctg cacaaccact acacgcagaa gagcctctcc 1380

ctgtctccgg gtaaa 1395

<210> 6
<211> 465
<212> PRT
<213> Artificial

<220>
<223> Chi-83D4 Heavy Chain

<400> 6

Met Glu Trp Arg Trp Val Phe Leu Phe Phe Leu Ser Val Thr Thr Gly
1               5                   10                  15

Val His Ser Gln Val Gln Leu Gln Gln Ser Asp Ala Glu Leu Val Lys
            20                  25                  30

Pro Gly Ala Ser Val Lys Ile Ser Cys Lys Ala Ser Gly Tyr Thr Phe
        35                  40                  45

Thr Asp His Ala Ile His Trp Val Lys Gln Lys Pro Glu Gln Gly Leu
    50                  55                  60

Glu Trp Ile Gly Tyr Phe Ser Pro Gly Asn Gly Asp Ile Lys Tyr Asn
65                  70                  75                  80

Glu Lys Phe Lys Gly Lys Ala Thr Leu Thr Ala Asp Lys Ser Ser Ser
                85                  90                  95

Thr Ala Tyr Met Gln Leu Asn Ser Leu Thr Ser Glu Asp Ser Ala Val
            100                 105                 110

Tyr Phe Cys Lys Arg Ser Tyr Gly Asn Tyr Asp Tyr Trp Gly Gln Gly
        115                 120                 125

Thr Thr Leu Thr Val Ser Ser Ala Ser Thr Lys Gly Pro Ser Val Phe
    130                 135                 140

Pro Leu Ala Pro Ser Ser Lys Ser Thr Ser Gly Gly Thr Ala Ala Leu
145                 150                 155                 160

Gly Cys Leu Val Lys Asp Tyr Phe Pro Glu Pro Val Thr Val Ser Trp
                165                 170                 175

Asn Ser Gly Ala Leu Thr Ser Gly Val His Thr Phe Pro Ala Val Leu
            180                 185                 190

Gln Ser Ser Gly Leu Tyr Ser Leu Ser Ser Val Val Thr Val Pro Ser
        195                 200                 205

Ser Ser Leu Gly Thr Gln Thr Tyr Ile Cys Asn Val Asn His Lys Pro
    210                 215                 220

Ser Asn Thr Lys Val Asp Lys Lys Val Glu Pro Lys Ser Cys Asp Lys
225                 230                 235                 240

Thr His Thr Cys Pro Pro Cys Pro Ala Pro Glu Leu Leu Gly Gly Pro
                245                 250                 255

Ser Val Phe Leu Phe Pro Pro Lys Pro Lys Asp Thr Leu Met Ile Ser
            260                 265                 270

Arg Thr Pro Glu Val Thr Cys Val Val Val Asp Val Ser His Glu Asp
        275                 280                 285

Pro Glu Val Lys Phe Asn Trp Tyr Val Asp Gly Val Glu Val His Asn
    290                 295                 300

Ala Lys Thr Lys Pro Arg Glu Glu Gln Tyr Asn Ser Thr Tyr Arg Val
305                 310                 315                 320

Val Ser Val Leu Thr Val Leu His Gln Asp Trp Leu Asn Gly Lys Glu
                325                 330                 335

Tyr Lys Cys Lys Val Ser Asn Lys Ala Leu Pro Ala Pro Ile Glu Lys
            340                 345                 350

Thr Ile Ser Lys Ala Lys Gly Gln Pro Arg Glu Pro Gln Val Tyr Thr
        355                 360                 365

Leu Pro Pro Ser Arg Asp Glu Leu Thr Lys Asn Gln Val Ser Leu Thr

370                 375                 380

Cys Leu Val Lys Gly Phe Tyr Pro Ser Asp Ile Ala Val Glu Trp Glu
385                 390                 395                 400

Ser Asn Gly Gln Pro Glu Asn Asn Tyr Lys Thr Thr Pro Pro Val Leu
                405                 410                 415

Asp Ser Asp Gly Ser Phe Phe Leu Tyr Ser Lys Leu Thr Val Asp Lys
            420                 425                 430

Ser Arg Trp Gln Gln Gly Asn Val Phe Ser Cys Ser Val Met His Glu
        435                 440                 445

Ala Leu His Asn His Tyr Thr Gln Lys Ser Leu Ser Leu Ser Pro Gly
    450                 455                 460

Lys
465

<210>  7
<211>  702
<212>  DNA
<213>  Artificial

<220>
<223>  Chi-83D4 Light Chain complete cDNA

<220>
<221>  misc_feature
<222>  (1)..(381)
<223>  VL region

<220>
<221>  misc_feature
<222>  (130)..(162)
<223>  L-CDR1

<220>
<221>  misc_feature
<222>  (208)..(228)
<223>  L-CDR2

<220>
<221>  misc_feature
<222>  (325)..(351)
<223>  L-CDR3

<400>  7
atggtatcca cacctcagtt ccttgtattt ttgcttttct ggattccagc ctccagaggt      60

gacatcttgc tgactcagtc tccagccatc ctgtctgtga gtccaggaga aagagtcagt     120

ttctcctgca gggccagtca gaacattggc acaagtatac actggtatca gcaaagaaca 180

aatggttctc caaggcttct cataaagtat gcttctgagt ctgtctctgg gatcccttcc 240

aggtttagtg gcagtggatc agggacagat tttactctta gcatcaacag tgtggagtct 300

gaagatattg cagattatta ctgtcaacat actaatagct ggccaaccac gttcggaggg 360

gggaccaaac tggaaataaa acgaactgtg gctgcaccat ctgtcttcat cttcccgcca 420

tctgatgagc agttgaaatc tggaactgcc tctgttgtgt gcctgctgaa taacttctat 480

cccagagagg ccaaagtaca gtggaaggtg gataacgccc tccaatcggg taactcccag 540

gagagtgtca cagagcagga cagcaaggac agcacctaca gcctcagcag caccctgacg 600

ctgagcaaag cagactacga gaaacacaaa gtctacgcct gcgaagtcac ccatcagggc 660

ctgagctcgc ccgtcacaaa gagcttcaac aggggagagt gt 702

<210> 8
<211> 234
<212> PRT
<213> Artificial

<220>
<223> Chi-83D4 Light Chain

<400> 8

Met Val Ser Thr Pro Gln Phe Leu Val Phe Leu Leu Phe Trp Ile Pro
1               5                   10                  15

Ala Ser Arg Gly Asp Ile Leu Leu Thr Gln Ser Pro Ala Ile Leu Ser
            20                  25                  30

Val Ser Pro Gly Glu Arg Val Ser Phe Ser Cys Arg Ala Ser Gln Asn
        35                  40                  45

Ile Gly Thr Ser Ile His Trp Tyr Gln Gln Arg Thr Asn Gly Ser Pro
    50                  55                  60

Arg Leu Leu Ile Lys Tyr Ala Ser Glu Ser Val Ser Gly Ile Pro Ser
65                  70                  75                  80

Arg Phe Ser Gly Ser Gly Ser Gly Thr Asp Phe Thr Leu Ser Ile Asn
                85                  90                  95

Ser Val Glu Ser Glu Asp Ile Ala Asp Tyr Tyr Cys Gln His Thr Asn
            100                 105                 110

Ser Trp Pro Thr Thr Phe Gly Gly Gly Thr Lys Leu Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210>  9
<211>  22
<212>  DNA
<213>  Artificial

<220>
<223>  primer

<400>  9
aggtsmarct gcagsagtcw gg                                              22

<210>  10
<211>  32
<212>  DNA
<213>  Artificial

<220>
<223>  primer

<400>  10
tgaggagacg gtgaccgtgg tcccttggcc cc                                   32

<210>  11
<211>  24
<212>  DNA
<213>  Artificial

<220>
<223>  primer

<400>  11
gacattgagc tcacccagtc tcca                                              24


<210>  12
<211>  24
<212>  DNA
<213>  Artificial

<220>
<223>  primer

<400>  12
ccgtttgatt tccagcttgg tgcc                                              24


<210>  13
<211>  24
<212>  DNA
<213>  Artificial

<220>
<223>  primer

<400>  13
ccgttttatt tccagcttgg tccc                                              24


<210>  14
<211>  24
<212>  DNA
<213>  Artificial

<220>
<223>  primer

<400>  14
ccgttttatt tccaactttg tccc                                              24


<210>  15
<211>  24
<212>  DNA
<213>  Artificial

<220>
<223>  primer

<400>  15
ccgtttcagc tccagcttgg tccc                                              24


<210>  16
<211>  46
<212>  DNA
<213>  Artificial

<220>
<223>  primer

<400>  16
tactcgcggc ccagccggcc atggcccagg tsmarctgca gsagtc                    46


<210>  17
<211>  29
<212>  DNA
<213>  Artificial

<220>
<223>  primer

<400>  17
ccgctcgaga ctgaggagac ggtgaccgt                                       29


<210>  18
<211>  39
<212>  DNA
<213>  Artificial

<220>
<223>  primer

<400>  18
accgcctcca ccagtgcaca ggacattgag ctcacccag                            39


<210>  19
<211>  42
<212>  DNA
<213>  Artificial

<220>
<223>  primer

<400>  19
ttttcctttt gcggccgccc gtttgatttc cagcttggtg cc                        42


<210>  20
<211>  42
<212>  DNA
<213>  Artificial

<220>
<223>  primer

<400>  20
ttttcctttt gcggccgccc gttttatttc cagcttggtc cc                        42


<210>  21
<211>  42
<212>  DNA
<213>  Artificial

<220>
<223>  primer

<400>  21

ttttcctttt gcggccgccc gttttatttc caactttgtc cc                    42

<210>  22
<211>  42
<212>  DNA
<213>  Artificial

<220>
<223>  primer

<400>  22
ttttcctttt gcggccgccc gtttcagctc cagcttggtc cc                    42

<210>  23
<211>  1419
<212>  DNA
<213>  Artificial

<220>
<223>  83D4scFv-hFc2

<220>
<221>  CDS
<222>  (1)..(1419)

<220>
<221>  misc_feature
<222>  (1)..(732)
<223>  83D4scFv

<400>  23
atg gcc cag gtg caa ctg cag cag tct ggc gct gag ttg gtg aaa cct      48
Met Ala Gln Val Gln Leu Gln Gln Ser Gly Ala Glu Leu Val Lys Pro
1               5                   10                  15

ggg gct tca gtg aag ata tcc tgc aag gct tct ggc tac acc ttc act      96
Gly Ala Ser Val Lys Ile Ser Cys Lys Ala Ser Gly Tyr Thr Phe Thr
            20                  25                  30

gac cat gct att cac tgg gtg aag cag aag cct gaa cag ggc ctg gaa      144
Asp His Ala Ile His Trp Val Lys Gln Lys Pro Glu Gln Gly Leu Glu
        35                  40                  45

tgg att gga tat ttt tcc ccc gga aat ggt gat att aag tac aat gag      192
Trp Ile Gly Tyr Phe Ser Pro Gly Asn Gly Asp Ile Lys Tyr Asn Glu
    50                  55                  60

aag ttc aag ggc aag gcc aca ctg act gca gac aaa tcc tcc agc act      240
Lys Phe Lys Gly Lys Ala Thr Leu Thr Ala Asp Lys Ser Ser Ser Thr
65                  70                  75                  80

gcc tac atg cag ctc aac agc ctg aca tct gag gat tct gct gtg tat      288
Ala Tyr Met Gln Leu Asn Ser Leu Thr Ser Glu Asp Ser Ala Val Tyr
                85                  90                  95

ttc tgt aaa aga tcc tat ggt aac tac gac tac tgg ggc caa ggg acc      336
Phe Cys Lys Arg Ser Tyr Gly Asn Tyr Asp Tyr Trp Gly Gln Gly Thr
            100                 105                 110

acg gtc acc gtc tcc tca gtc tcg agt ggt gga ggc ggt tca ggc gga 384
Thr Val Thr Val Ser Ser Val Ser Ser Gly Gly Gly Gly Ser Gly Gly
        115                 120                 125

ggt ggc tct ggc ggt agt gca cag gac att gag ctc acc cag tct cca 432
Gly Gly Ser Gly Gly Ser Ala Gln Asp Ile Glu Leu Thr Gln Ser Pro
    130                 135                 140

gcc atc ctg tct gtg agt cca gga gaa aga gtc agt ttc tcc tgc agg 480
Ala Ile Leu Ser Val Ser Pro Gly Glu Arg Val Ser Phe Ser Cys Arg
145                 150                 155                 160

gcc agt cag aac att ggc aca agt ata cac tgg tat cag caa aga aca 528
Ala Ser Gln Asn Ile Gly Thr Ser Ile His Trp Tyr Gln Gln Arg Thr
                165                 170                 175

aat ggt tct cca agg ctt ctc ata aag tat gcc tct gag tct gtc tct 576
Asn Gly Ser Pro Arg Leu Leu Ile Lys Tyr Ala Ser Glu Ser Val Ser
            180                 185                 190

ggg atc cct tcc agg ttt agt ggc agt gga tca ggg aca gat ttt act 624
Gly Ile Pro Ser Arg Phe Ser Gly Ser Gly Ser Gly Thr Asp Phe Thr
        195                 200                 205

ctt agc atc aac agt gtg gag tct gaa gat att gca gat tat tac tgt 672
Leu Ser Ile Asn Ser Val Glu Ser Glu Asp Ile Ala Asp Tyr Tyr Cys
    210                 215                 220

caa cat act aat agc tgg cca acc acg ttc gga ggg ggg acc aag ctg 720
Gln His Thr Asn Ser Trp Pro Thr Thr Phe Gly Gly Gly Thr Lys Leu
225                 230                 235                 240

gaa ata aaa cgg gcg gcc gct aga tct gtg gag tgc cca cct tgc cca 768
Glu Ile Lys Arg Ala Ala Ala Arg Ser Val Glu Cys Pro Pro Cys Pro
                245                 250                 255

gca cca cct gtg gca gga cct tca gtc ttc ctc ttc ccc cca aaa ccc 816
Ala Pro Pro Val Ala Gly Pro Ser Val Phe Leu Phe Pro Pro Lys Pro
            260                 265                 270

aag gac acc ctg atg atc tcc aga acc cct gag gtc acg tgc gtg gtg 864
Lys Asp Thr Leu Met Ile Ser Arg Thr Pro Glu Val Thr Cys Val Val
        275                 280                 285

gtg gac gtg agc cac gaa gac ccc gag gtc cag ttc aac tgg tac gtg 912
Val Asp Val Ser His Glu Asp Pro Glu Val Gln Phe Asn Trp Tyr Val
    290                 295                 300

gac ggc atg gag gtg cat aat gcc aag aca aag cca cgg gag gag cag 960
Asp Gly Met Glu Val His Asn Ala Lys Thr Lys Pro Arg Glu Glu Gln
305                 310                 315                 320

ttc aac agc acg ttc cgt gtg gtc agc gtc ctc acc gtc gtg cac cag 1008
Phe Asn Ser Thr Phe Arg Val Val Ser Val Leu Thr Val Val His Gln
                325                 330                 335

gac tgg ctg aac ggc aag gag tac aag tgc aag gtc tcc aac aaa ggc 1056
Asp Trp Leu Asn Gly Lys Glu Tyr Lys Cys Lys Val Ser Asn Lys Gly
            340                 345                 350

ctc cca gcc ccc atc gag aaa acc atc tcc aaa acc aaa ggg cag ccc     1104
Leu Pro Ala Pro Ile Glu Lys Thr Ile Ser Lys Thr Lys Gly Gln Pro
        355                 360                 365

cga gaa cca cag gtg tac acc ctg ccc cca tcc cgg gag gag atg acc     1152
Arg Glu Pro Gln Val Tyr Thr Leu Pro Pro Ser Arg Glu Glu Met Thr
    370                 375                 380

aag aac cag gtc agc ctg acc tgc ctg gtc aaa ggc ttc tac ccc agc     1200
Lys Asn Gln Val Ser Leu Thr Cys Leu Val Lys Gly Phe Tyr Pro Ser
385                 390                 395                 400

gac atc gcc gtg gag tgg gag agc aat ggg cag ccg gag aac aac tac     1248
Asp Ile Ala Val Glu Trp Glu Ser Asn Gly Gln Pro Glu Asn Asn Tyr
                405                 410                 415

aag acc aca cct ccc atg ctg gac tcc gac ggc tcc ttc ttc ctc tac     1296
Lys Thr Thr Pro Pro Met Leu Asp Ser Asp Gly Ser Phe Phe Leu Tyr
            420                 425                 430

agc aag ctc acc gtg gac aag agc agg tgg cag cag ggg aac gtc ttc     1344
Ser Lys Leu Thr Val Asp Lys Ser Arg Trp Gln Gln Gly Asn Val Phe
        435                 440                 445

tca tgc tcc gtg atg cat gag gct ctg cac aac cac tac aca cag aag     1392
Ser Cys Ser Val Met His Glu Ala Leu His Asn His Tyr Thr Gln Lys
    450                 455                 460

agc ctc tcc ctg tct ccg ggt aaa tga                                 1419
Ser Leu Ser Leu Ser Pro Gly Lys
465                 470


<210>  24
<211>  472
<212>  PRT
<213>  Artificial

<220>
<223>  Synthetic Construct

<400>  24

Met Ala Gln Val Gln Leu Gln Gln Ser Gly Ala Glu Leu Val Lys Pro
1               5                   10                  15


Gly Ala Ser Val Lys Ile Ser Cys Lys Ala Ser Gly Tyr Thr Phe Thr
            20                  25                  30


Asp His Ala Ile His Trp Val Lys Gln Lys Pro Glu Gln Gly Leu Glu
        35                  40                  45


Trp Ile Gly Tyr Phe Ser Pro Gly Asn Gly Asp Ile Lys Tyr Asn Glu
    50                  55                  60


Lys Phe Lys Gly Lys Ala Thr Leu Thr Ala Asp Lys Ser Ser Ser Thr
65                  70                  75                  80

Ala Tyr Met Gln Leu Asn Ser Leu Thr Ser Glu Asp Ser Ala Val Tyr
                85                  90                  95

Phe Cys Lys Arg Ser Tyr Gly Asn Tyr Asp Tyr Trp Gly Gln Gly Thr
            100                 105                 110

Thr Val Thr Val Ser Ser Val Ser Ser Gly Gly Gly Gly Ser Gly Gly
        115                 120                 125

Gly Gly Ser Gly Gly Ser Ala Gln Asp Ile Glu Leu Thr Gln Ser Pro
    130                 135                 140

Ala Ile Leu Ser Val Ser Pro Gly Glu Arg Val Ser Phe Ser Cys Arg
145                 150                 155                 160

Ala Ser Gln Asn Ile Gly Thr Ser Ile His Trp Tyr Gln Gln Arg Thr
                165                 170                 175

Asn Gly Ser Pro Arg Leu Leu Ile Lys Tyr Ala Ser Glu Ser Val Ser
            180                 185                 190

Gly Ile Pro Ser Arg Phe Ser Gly Ser Gly Ser Gly Thr Asp Phe Thr
        195                 200                 205

Leu Ser Ile Asn Ser Val Glu Ser Glu Asp Ile Ala Asp Tyr Tyr Cys
    210                 215                 220

Gln His Thr Asn Ser Trp Pro Thr Thr Phe Gly Gly Gly Thr Lys Leu
225                 230                 235                 240

Glu Ile Lys Arg Ala Ala Ala Arg Ser Val Glu Cys Pro Pro Cys Pro
                245                 250                 255

Ala Pro Pro Val Ala Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser His Glu Asp Pro Glu Val Gln Phe Asn Trp Tyr Val
    290                 295                 300

Asp Gly Met Glu Val His Asn Ala Lys Thr Lys Pro Arg Glu Glu Gln
305                 310                 315                 320

Phe Asn Ser Thr Phe Arg Val Val Ser Val Leu Thr Val Val His Gln
                325                 330                 335

Asp Trp Leu Asn Gly Lys Glu Tyr Lys Cys Lys Val Ser Asn Lys Gly
            340                 345                 350

Leu Pro Ala Pro Ile Glu Lys Thr Ile Ser Lys Thr Lys Gly Gln Pro
        355                 360                 365

Arg Glu Pro Gln Val Tyr Thr Leu Pro Pro Ser Arg Glu Glu Met Thr
    370                 375                 380

Lys Asn Gln Val Ser Leu Thr Cys Leu Val Lys Gly Phe Tyr Pro Ser
385                 390                 395                 400

Asp Ile Ala Val Glu Trp Glu Ser Asn Gly Gln Pro Glu Asn Asn Tyr
                405                 410                 415

Lys Thr Thr Pro Pro Met Leu Asp Ser Asp Gly Ser Phe Phe Leu Tyr
            420                 425                 430

Ser Lys Leu Thr Val Asp Lys Ser Arg Trp Gln Gln Gly Asn Val Phe
        435                 440                 445

Ser Cys Ser Val Met His Glu Ala Leu His Asn His Tyr Thr Gln Lys
    450                 455                 460

Ser Leu Ser Leu Ser Pro Gly Lys
465                 470


<210>  25
<211>  11
<212>  PRT
<213>  Artificial

<220>
<223>  L-CDR1

<400>  25

Arg Ala Ser Gln Asn Ile Gly Thr Ser Ile His
1               5                   10


<210>  26
<211>  7
<212>  PRT
<213>  Artificial

<220>

<223>  L-CDR2

<400>  26

Tyr Ala Ser Glu Ser Val Ser
1               5


<210>  27
<211>  9
<212>  PRT
<213>  Artificial

<220>
<223>  L-CDR3

<400>  27

Gln His Thr Asn Ser Trp Pro Thr Thr
1               5


<210>  28
<211>  5
<212>  PRT
<213>  Artificial

<220>
<223>  H-CDR1

<400>  28

Asp His Ala Ile His
1               5


<210>  29
<211>  17
<212>  PRT
<213>  Artificial

<220>
<223>  H-CDR2

<400>  29

Tyr Phe Ser Pro Gly Asn Gly Asp Ile Lys Tyr Asn Glu Lys Phe Lys
1               5                   10                  15


Gly


<210>  30
<211>  7
<212>  PRT
<213>  Artificial

<220>
<223>  H-CDR3

<400>  30

Ser Tyr Gly Asn Tyr Asp Tyr
1               5